# EUROPEAN PATENT SPECIFICATION

(11) **EP 4 541 259 B1**
(45) Date of publication and mention of the grant of the patent: **05.08.2026**
(21) Application number: 22949739.1
(22) Date of filing: 05.07.2022
(51) Int. Cl.: A61N 5/06, A61B 5/00

(54) **IMPLANTABLE FLEXIBLE PHOTOELECTRODE DEVICE AND BRAIN-COMPUTER INTERFACE APPARATUS THEREOF**
IMPLANTIERBARE FLEXIBLE FOTOELEKTRODENVORRICHTUNG UND GEHIRN-COMPUTER-SCHNITTSTELLENVORRICHTUNG DAFÜR
DISPOSITIF DE PHOTOÉLECTRODE FLEXIBLE IMPLANTABLE ET APPAREIL D'INTERFACE CERVEAU-ORDINATEUR ASSOCIÉ

(43) Date of publication of application: 23.04.2025
(73) Proprietor: Boe Technology Group Co., Ltd., Beijing 100015 (CN)
(72) Inventor: JIANG, Xiaotian, Beijing 100176 (CN); CHEN, Yanshun, Beijing 100176 (CN); LI, Xinguo, Beijing 100176 (CN); WU, Xinyin, Beijing 100176 (CN); ZHANG, Honglei, Beijing 100176 (CN); ZHANG, Xiying, Beijing 100176 (CN)
(74) Representative: Klunker IP Patentanwälte PartG mbB
(86) International application number: PCT/CN2022/103903
(87) International publication number: WO 2024/007151

(56) References cited:
- CN-A- 113 268 142
- CN-A- 113 795 189
- CN-U- 209 004 012
- US-A1- 2013 046 148
- US-A1- 2015 047 179
- US-A1- 2020 163 696
- GONCALVES S B ET AL: "Design and manufacturing challenges of optogenetic neural interfaces: a review", JOURNAL OF NEURAL ENGINEERING, INSTITUTE OF PHYSICS PUBLISHING, BRISTOL, GB, vol. 14, no. 4, 19 May 2017 (2017-05-19), pages 41001, XP020318475, ISSN: 1741-2552, [retrieved on 20170519], DOI: 10.1088/1741-2552/AA7004

## Description

### TECHNICAL FIELD

The present invention relates to the field of medical instruments, in particular to an implantable flexible photoelectrode device and a brain-computer interface apparatus thereof.

### BACKGROUND

Optogenetic technology, as one of neural regulation methods, have received increasing attention. Electrical or drug stimulation is to stimulate target neurons by using electricity or a drug. Therefore, a brain-computer interface apparatus based on optogenetic technology have a wide range of uses in medical, entertainment industries, and the like.

] US2015/047179A1 reviews the design and manufacturing challenges of optogenetic neural interfaces, focusing on optical tools for cell-type specific neuromodulation.

US2013/046148A1 discloses a three-dimensional penetrating optical-electrical neural interface array for selective stimulation and recording. The article "Design and manufacturing challenges of optogenetic neural interfaces: a review" describes a waveguide neural interface device that combines light delivery with electrical recording for targeted tissue illumination.

### SUMMARY

It is an object of the present invention to provide an implantable flexible photoelectrode device and a brain-computer interface apparatus thereof.

The object is achieved by the features of the respective independent claims. Further embodiments are defined in the corresponding dependent claims. Even though the description refers to embodiments or to the invention, it is to be understood that the invention is defined by the claims and embodiments of the invention are those comprising at least all the features of one of the independent claims.

In some embodiments, the first light-shielding layer comprises a first light-transmitting part, the first light-transmitting part is configured to transmit the light beam therethrough to exit in a direction away from the light emitting region.

**In** some embodiments, the first light-transmitting part comprises a first via hole in the first light-shielding layer, an orthographic projection of the first via hole on the first flexible support layer is located within an orthographic projection of the light emitting region on the first flexible support layer.

**In** some embodiments, the first via hole is plural, orthographic projections of part of a plurality of the first via holes on the first flexible support layer are located within the orthographic projection of the light emitting region on the first flexible support layer, and orthographic projections of another part of the plurality of the first via holes on the first flexible support layer are located outside the orthographic projection of the light emitting region on the first flexible support layer.

In some embodiments, a planar shape of the first via hole is at least one of a square, a circle, an ellipse, a rectangle, and a parallelogram.

In some embodiments, a size of the first via hole is 0.1-99% of a size of the light emitting region.

In some embodiments, an area of the first via hole is 1-225 µm², and an area of the light emitting region N is 400-900 µm².

In some embodiments, the light emitting unit is plural, and a plurality of the light emitting units have a plurality of light emitting regions; the first light-shielding layer comprises a plurality of first light-transmitting parts, and the plurality of the first light-transmitting parts are disposed in one-to-one correspondence with the plurality of the light emitting regions.

In some embodiments, the plurality of the light emitting regions are disposed without a gap therebetween, such that the plurality of the light emitting regions are connected to each other.

In some embodiments, the first electrode and the second electrode overlap each other in a thickness direction perpendicular to the second flexible support layer.

In some embodiments, the light emitting unit is plural, the first electrode is plural, the second electrode is plural, a plurality of the first electrodes and a plurality of the second electrodes are arranged in one-to-one correspondence and constitute a plurality of electrode groups; wherein each light emitting unit is arranged in one-to-one correspondence with one of the plurality of the electrode groups.

In some embodiments, the implantable flexible photoelectrode device further comprises: a fixing assembly, the fixing assembly being located on the main body and configured to fix the main body in place.

In some embodiments, the fixing assembly comprises: an adhesive; a storage container, configured to store the adhesive; wherein the main body has a circumferential direction perpendicular to a direction of an extension direction of the main body, the storage container being arranged along the circumferential direction of the main body.

In some embodiments, the main body comprises a first end away from the light emitting assembly and a second end close to the light emitting assembly along the extending direction, the storage container is located on the first end of the main body.

In some embodiments, the storage container is configured to automatically release the adhesive when the implantable flexible photoelectrode device is fixed in place.

In some embodiments, the implantable flexible photoelectrode device is configured to be implanted at a position below cranial bones of a brain and the light irradiating region is a region of cerebral cortex within the brain.

According to a second aspect of the present disclosure, it is provided a brain-computer interface apparatus, comprising the above implantable flexible photoelectrode device.

In some embodiments, the brain-computer interface apparatus further comprises: a housing; and a control device, the control device is in the housing and connected to the implantable flexible photoelectrode device, the control device being configured to be in signal communication with the implantable flexible photoelectrode device; wherein the main body of the implantable flexible photoelectrode device is configured to move with the housing and the control device.

In some embodiments, the implantable flexible photoelectrode device further comprises an electrode assembly, wherein the control device comprises: a central control module; a drive module, one end of the drive module being connected with the central control module, and the other end of the drive module being connected with the light emitting assembly of the implantable flexible photoelectrode device and configured to provide a drive signal to the light emitting assembly; and a detection module, one end of the detection module being connected with the central control module, and the other end of the detection module being connected with the electrode assembly of the implantable flexible photoelectrode device and configured to receiving a signal from the electrode assembly.

In some embodiments, the brain-computer interface apparatus further comprises: a power supply module, connected to the central control module, the drive module, and the detection module and configured to supply power, wherein the power supply module is located in the housing.

In some embodiments, the central control module comprises: a control circuit, configured to generate a drive signal; and a communication circuit connected to the control circuit, a first end of the communication circuit being connected to the drive module and a second end being connected to the detection module, the communication circuit being configured to transmit the drive signal to the drive module and to transmit the signal from the electrode assembly provided by the detection module to the control circuit.

In some embodiments, the housing has a longitudinal axis, the housing comprises a first size along the longitudinal axis and a second size perpendicular to the longitudinal axis, the first size is smaller than the second size.

In some embodiments, the housing has a circumferential direction perpendicular to the longitudinal axis, the implantable flexible photoelectrode device is plural, a plurality of the implantable flexible photoelectrode devices are distributed along the circumferential direction.

In some embodiments, the housing comprises a first side and a second side oppositely disposed in the longitudinal axis, the main body of the implantable flexible photoelectrode device is connected to at least one of the first side and the second side.

In some embodiments, the brain-computer interface apparatus further comprises: a fixing component, the fixing component and the main body being located on the same side of the housing, and the fixing component being configured to fix the brain-computer interface apparatus in place.

In some embodiments, the fixing component comprises an adhesive.

In some embodiments, a part or an entirety of the brain-computer interface apparatus is configured to be implantable.

In some embodiments, the flexible photoelectrode device is configured to be implanted below cranial bones of a brain, and the housing is configured to be implanted at and/or below the cranial bones of the brain.

### BRIEF DESCRIPTION OF DRAWINGS

In order to more clearly illustrate the technical solutions of the present invention, the accompanying drawings will be briefly introduced below.
FIG. 1 is a structural schematic diagram of a brain-computer interface apparatus;
FIG. 2 is a schematic plan view of an implantable photoelectrode device in FIG. 1;
FIG. 3 is a cross-sectional view taken along line I-I of FIG. 1;
FIG. 4 is a structural schematic diagram of a brain-computer interface apparatus
FIG. 5 is a block diagram showing connections of various components of a brain-computer interface apparatus provided by an embodiment of the present invention;
FIG. 6 is a partially enlarged schematic diagram of a first connecting part of FIG. 4;
FIG. 7 is a schematic cross-sectional view taken along line AA of FIG. 6;
FIG. 8 is a structural schematic diagram of a light emitting unit provided by an embodiment of the present invention;
FIG. 9 is a structural schematic diagram of a light emitting unit provided by another embodiment of the present invention;
FIG. 10 is a partial plan view of a light emitting region and a first via hole provided by an embodiment of the present invention;
FIG. 11 is a partial plan view of a light emitting region and a first via hole provided by another embodiment of the present invention;
FIG. 12 is a cross-sectional view of a first electrode and a light emitting unit;
FIG. 13 is a structural schematic diagram of a first electrode and a second electrode;
FIG. 14 is a schematic cross-sectional view of a fixing assembly 13 of FIG. 4;
FIG. 15 is a block diagram of a brain-computer interface apparatus provided by another embodiment of the present invention;
FIG. 16 is a partially enlarged schematic diagram of a first connecting part of FIG. 15;
FIG. 17 is a schematic cross-sectional view taken along line BB of FIG. 16;
FIG. 18 is a partially enlarged schematic diagram of a second connecting part of FIG. 15;
FIG. 19 is a schematic cross-sectional view taken along line CC of FIG. 18;
FIG. 20 is a partial cross-sectional view of a first connecting part and a second connecting part provided by an embodiment of the present invention;
FIG. 21 is a front view of a housing of FIG. 4;
FIG. 22 is a cross-sectional view taken along line DD of FIG. 21;
FIG. 23 is a block diagram of a brain-computer interface apparatus;
FIG. 24 is a block diagram of a brain-computer interface apparatus;
FIG. 25 is a block diagram of a brain-computer interface apparatus.

### DETAILED DESCRIPTION

In order to make the objects, technical solutions and advantages of the present invention clearer, the technical solutions of the present invention will be clearly and completely described below in conjunction with the accompanying drawings.

Unless defined otherwise, technical or scientific terms used herein are to be given their ordinary meaning as understood by an ordinary skilled in the art to which this disclosure belongs. The terms "first," "second," and the like as used in the description and claims of the disclosed patent application do not denote any order, quantity, or importance, but rather are used merely to distinguish one component from another. Words "comprising" or "includes" and the like are intended to mean that elements or items preceding "comprising" or "includes" now encompass the elements or items listed following "comprising" or "includes" and do not exclude other elements or items. The terms "connect" or "connected" and the like are not restricted to physical or mechanical connections, but may include electrical connections, whether direct or indirect. "Upper", "lower", "left", "right" and the like are merely used to indicate relative positional relationships, which may change accordingly when the absolute position of the object being described changes.

Typically, a brain-computer interface apparatus based on optogenetic technology includes a photoelectrode device which may include a light emitting assembly (also referred to as light supplying assembly) for emitting light for irradiation and an electrode assembly for recording electrical signals. Due to the absorption or scattering effect of the light by living organisms, the light generated outside an animal's brain cannot reach deep into the brain, much less accurately reach a specific area. Accordingly, an implantable photoelectrode device that can direct light onto a neuron is provided.

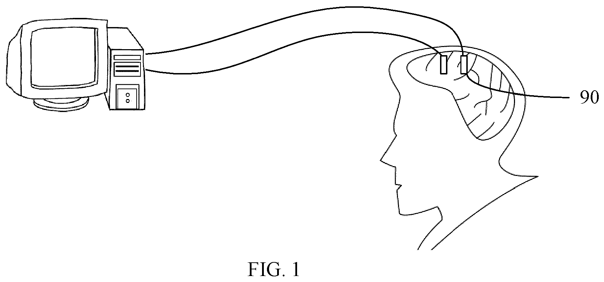

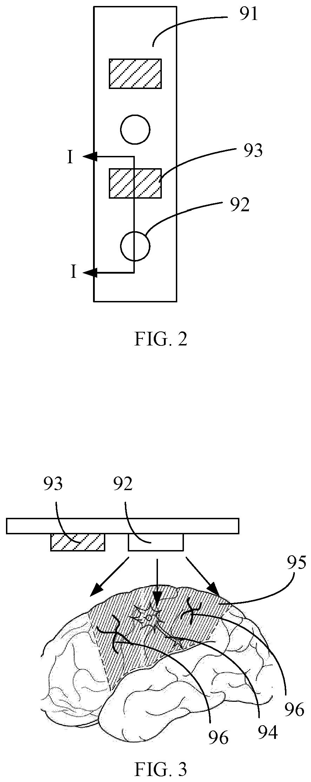

FIG. 1 is a block diagram of a brain-computer interface apparatus. FIG. 2 is a schematic plan view of an implantable photoelectrode device in FIG. 1, and FIG. 3 is a cross-sectional view taken along line I-I of FIG. 1.

As shown in FIGS. 1 to 3, the implantable photoelectrode device 90 includes a flexible layer 91 and a light emitting assembly and an electrode assembly located on the flexible layer 91; the light emitting assembly includes a plurality of light emitting units 92 and the electrode assembly includes a plurality of electrodes 93. The light beam emitted from each light emitting unit 92 is irradiated onto neuronal cells of the brain to form a light irradiating region 95. After stimulated by the light beam, the brain's neuronal cells generate bioelectric signals. The electrode 93 records and transmits the bioelectric signals to a computer at the back end.

As shown in FIG. 3, when it is desired to irradiate a specific neuronal cell 94, because the light beam emitted from the light emitting unit 92 has a large divergence angle, the area of the light irradiating region 95 (the shadow region in the drawing) is large, so that not only the target neuronal cell 94 but also the neuronal cells 96 in the vicinity thereof are irradiated. As a result, the optical path is dispersed, and precise control of the optical path cannot be achieved. Furthermore, because most of the signals recorded by the electrode assembly is in the order of microvolts, it is prone to be disturbed by the external environment; because the light beam also impinges on areas other than the target neuronal cell, it results in a reduction in the signal-to-noise ratio of the photoelectrode device.

To this end, the present disclosure provide an implantable flexible photoelectrode device and a brain-computer interface apparatus thereof to avoid optical path dispersion and improve the light modulation accuracy.

The present disclosure provides an implantable flexible photoelectrode device, including: a main body and a first connecting part connected with the main body, the first connecting part including a first flexible support layer, a light emitting assembly, and a first light-shielding layer. The light emitting assembly is located on the first flexible support layer and configured to provide a light beam to the light irradiating region. The light emitting assembly includes a light emitting unit, and the light emitting unit has a light emitting region and is configured to exit a light beam from the light emitting region, an orthographic projection of the light irradiating region on the first flexible support layer is located within an orthographic projection of the light emitting region on the first flexible support layer.

In the above-mentioned implantable flexible photoelectrode device, the area of the light irradiating region is reduced by setting the orthographic projection of the light irradiating region on the first flexible support layer to be within the orthographic projection of the light emitting region on the first flexible support layer. Compared to the case where the light irradiating region in FIG. 3 is relatively larger, the present disclosure solve the problem of optical path dispersion by reducing the area of the light irradiating region, so that the light beam is more easily to concentrate to a particular neuronal cell, thereby increasing the light modulation accuracy.

In embodiments of the present invention, the "light emitting region" refers to a region where the light emitting surface is located when the light beam exits from the light emitting unit. The "light irradiating region" refers to a region that is irradiated by the light beam emitted from the light emitting unit.

In embodiments of the present invention, the "implantable flexible photoelectrode device" refers to that the flexible photoelectrode device may be placed in a human body. For example, in some embodiments, the implantable flexible photoelectrode device is implanted at a position below cranial bones of the brain, and the light irradiating region is a cerebral cortex region of the brain.

In embodiments of the present invention, by disposing the first light-shielding layer on the light emitting unit, the light beam emitted from the light emitting unit may be partially blocked by the first light-shielding layer, thereby reducing the area of the light irradiating region.

The present invention will be illustrated below by using several specific examples. In order to keep the following description of the embodiments of the present invention clear and concise, detailed descriptions of known functions or components are omitted. When any component in an embodiment of the present invention appears in more than one figure, the component may be represented by the same reference numerals in each figure.

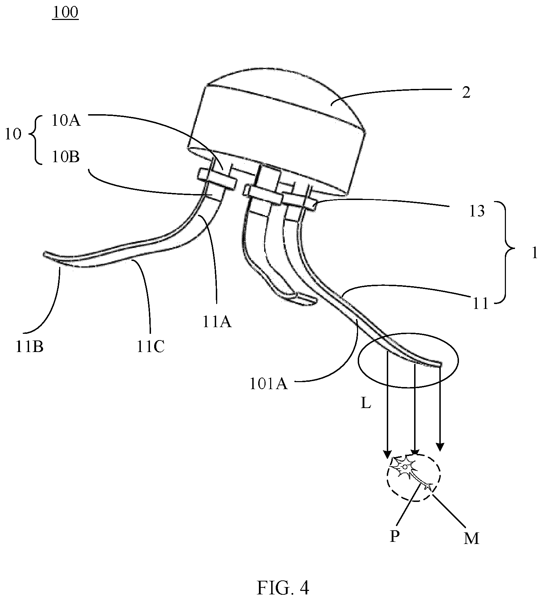

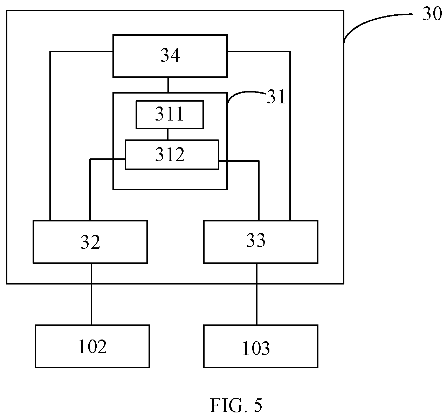

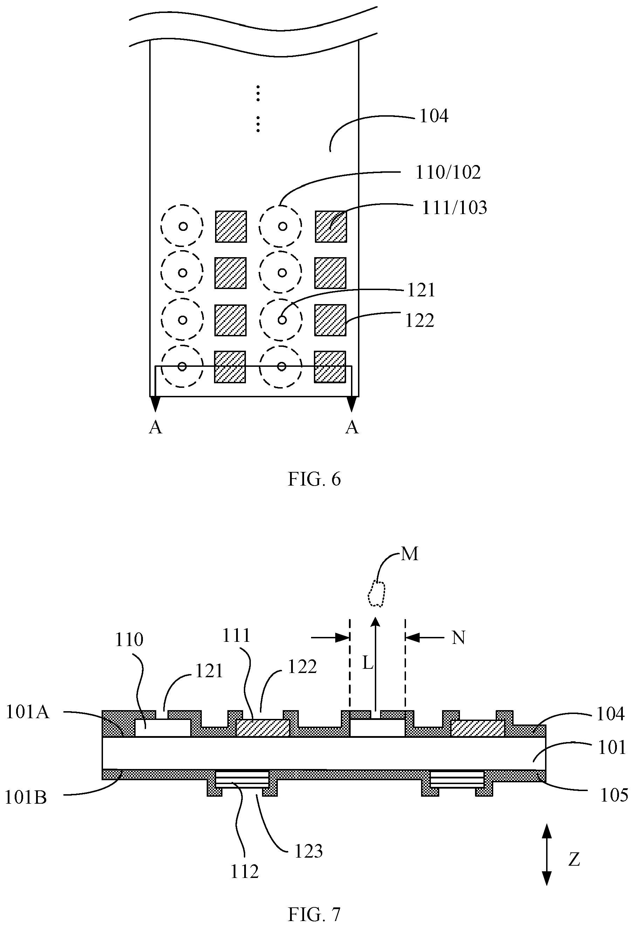

FIG. 4 is a structural schematic diagram of a brain-computer interface apparatus provided by the present disclosure. FIG. 5 is a block diagram showing connections of various components of a brain-computer interface apparatus provided by an embodiment of the present disclosure. FIG. 6 is a partially enlarged schematic view of a first connecting part of FIG. 4. FIG. 7 is a schematic cross-sectional view taken along line AA of FIG. 6.

As shown in FIG. 4, the brain-computer interface apparatus 100 provided by the present disclosure includes a plurality of implantable flexible photoelectrode devices 1, a housing 2 and a control device (not shown) located in the housing 2.

The implantable flexible photoelectrode device 1 includes a main body 10 and a first connecting part 11 connected with the main body 10. The main body 10 includes a first end 10A and a second end 10B oppositely arranged along an extending direction of the main body 10; the first end 10A is connected with the housing 2, the second end 10B is connected with the first connecting part 11.

As shown in FIGS. 6 and 7, the first connecting part 11 includes a first flexible support layer 101, a light emitting assembly 102, and a first light-shielding layer 104.

The light emitting assembly 102 is located on the first flexible support layer 101 and configured to provide a light beam L to a light irradiating region M (shown with reference to FIG. 4).

As shown in FIG. 7, the first flexible support layer 101 includes a first side 101A and a second side 101B oppositely arranged in a thickness direction perpendicular to the first flexible support layer 101 (e.g., the Z direction shown in the figure), and the light emitting assembly 102 is located on at least one of the first side 101A and the second side 101B. As shown in FIG. 4, when the target object P is located at the first side 101A of the first flexible support layer 101 (i.e., the lower side of the first connecting part 11 in FIG. 4), the light emitting assembly 102 is arranged at the first side 101A, such that the light beam L emitted by the light emitting assembly 102 can irradiate the target object P located in the light irradiating region M. When there are a plurality of target objects P distributed on both the first side 101A and the second side 101B of the first flexible support layer 101, each of the first side 101A and the second side 101B of the first flexible support layer 101 are provided with the light emitting assembly 102, so that the plurality of target objects P can be irradiated. Thus, by providing the light emitting assembly 102 on at least one of the first side 101A and the second side 101 B, the flexibility of the implantable flexible photoelectrode device 1 in different application scenarios can be increased and various light irradiating requirements can be met. The present disclosure are described with the light emitting assembly 102 being located on the only first side 101A.

As shown in FIGS. 6 and 7, the light emitting assembly 102 includes a light emitting unit 110 having a light emitting region N and configured to emit a light beam L from the light emitting region N. The orthographic projection of the light irradiating region M on the first flexible support layer 101 is set to be located within the orthographic projection of the light emitting region N on the first flexible support layer 101. In this way, by disposing the first light-shielding layer 104 on the light emitting unit 110, the light beam L emitted from the light emitting unit 110 is partially blocked by the first light-shielding layer 104, thereby reducing the area of the light irradiating region M. Compared with the case where the light irradiating region in FIG. 3 is relatively larger, the problem of optical path dispersion is solved by reducing the area of the light irradiating region M, so that the light beam is more easily to concentrate to a particular neuronal cell, thereby increasing the light modulation accuracy.

Embodiments of the present invention do not particularly limit the categories of the light emitting unit 110. In some embodiments, the light emitting unit 110 may adopt a light emitting diode device, such as a micro light emitting diode (Micro-LED) device or a quantum-dot light emitting diode (QLED) device. Micro-LEDs are preferred over QLEDs because of their high reliability and long lifetime.

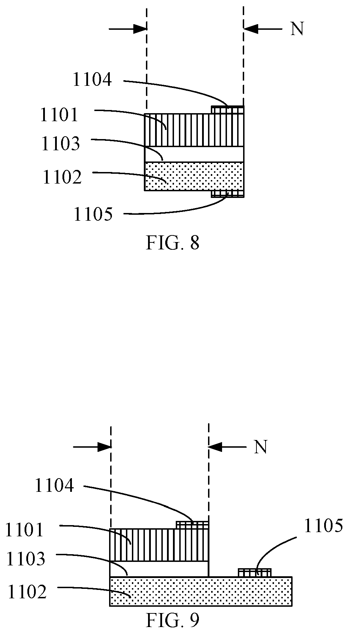

FIG. 8 is a structural schematic diagram of a light emitting unit provided by an embodiment of the present invention. As shown in FIG. 8, for example, the light emitting unit 110 is a Micro-LED die including a first semiconductor layer 1101 and a second semiconductor layer 1102 laminated together.

For example, the first semiconductor layer 1101 and the second semiconductor layer 1102 are semiconductor layers doped with different types of impurities, respectively. For example, one of the first semiconductor layer 1101 and the second semiconductor layer 1102 is a P-type semiconductor layer, for example, P-type GaN; the other is an N-type semiconductor layer, such as N-type GaN. When a voltage is applied between the P-type semiconductor layer and the N-type semiconductor layer, electrons and holes are recombined together, such that the monochromatic light is emitted.

As shown in FIG. 8, for example, the Micro-LED die further includes a semiconductor active layer 1103 disposed between the first semiconductor layer 1101 and the second semiconductor layer 1102. The semiconductor active layer 1103 is, for example, a quantum well layer.

As shown in FIG. 8, for example, the Micro-LED die further includes a first metal pad 1104 and a second metal pad 1105. The first metal pad 1104 is connected with the first semiconductor layer 1101 to provide a first electrical signal, e.g., a first voltage, to the first semiconductor layer 1101. The second metal pad 1105 is connected with the second semiconductor layer 1102 to provide a second electrical signal, for example, a second voltage to the second semiconductor layer 1102, and the first voltage and the second voltage are different from each other, thereby forming an electric field between the first semiconductor layer 1101 and the second semiconductor layer 1102.

FIG. 9 is a structural schematic diagram of a light emitting unit provided by another embodiment of the present invention. FIG. 9 differs from FIG. 8 in that the second semiconductor layer 1102 in FIG. 9 has a larger area than the first semiconductor layer 1101, that is, the orthographic projection of the first semiconductor layer 1101 on the second semiconductor layer 1102 is located in the region where the second semiconductor layer 1102 is located. With the above-mentioned structure, it is more convenient to provide the first metal pad 1104 and the second metal pad 1105 on the same side of the Micro-LED die (e.g., the upper side of the Micro-LED die shown in FIG. 9).

In some embodiments, the light emitting assembly 102 further includes a first power supply line and a second power supply line; the first metal pad 1104 is connected with the first power supply line and the second metal pad 1105 is connected with the second power supply line. When the first metal pad 1104 and the second metal pad 1105 are located on the same side of the Micro-LED die, the first power supply line and the second power supply line can also be located on the same side of the Micro-LED die, which can simplify the routing design of the first and second power supply lines and reduce the manufacturing difficulty.

In some embodiments, the first power supply line and the second power supply line are arranged in the first connecting part 11, for example at the first side 101A of the first flexible support layer 101. For example, the first power supply line and the second power supply line are extended from the first metal pad 1104 and the second metal pad 1105 respectively into the control device in the housing 2.

In embodiments of the present invention, the light emitting region N is an overlapping region of the first semiconductor layer 1101 and the second semiconductor layer 1102. For example, in FIGS. 8 and 9, the light emitting region N is an overlapping region of the first semiconductor layer 1101 and the second semiconductor layer 1102.

In some embodiments, at least part region of the first connecting part 11 is provided with the light emitting assembly 102. For example, as illustrated in FIG. 4, the first connecting part 11 includes, along the extending direction thereof, a first region 11A close to the main body 10, a second region 11B away from the main body 10, and a third region 11C located between the first region 11A and the second region 11B. The light emitting assembly 102 may be located in at least one of the first region 11A, the second region 11B, and the third region 11C. In this way, the position of the light emitting assembly 102 can be flexibly set according to actual needs. Embodiments of the present invention are described by taking the light emitting assembly 102 being located in the first region 11A, the second region 11B, and the third region 11C as an example.

As shown in FIG. 7, the first light-shielding layer 104 is located on a side of the light emitting unit 110 away from the first flexible support layer 101, and the first light-shielding layer 104 is configured to cover a portion of the light emitting region N so as to make the light beam L partially shielded by the first light-shielding layer 104.

By disposing the first light-shielding layer 104 on the light emitting unit 110, the light beam L emitted from the light emitting unit 104 is partially blocked by the first light-shielding layer 104, the divergence angle of the light beam L is reduced, and the collimation effect can be almost achieved, and therefore, the light beam L is more easily concentrated onto specific neuronal cells, thereby solving the problem of the light path dispersion, and increasing the light modulation accuracy.

Further, in FIG. 3, because the divergence angle of the light emitting units 92 is large, there may be an overlap of the light beams between adjacent light emitting units 92, and the target object in the overlap region may be irradiated with the superimposed light, resulting in a decrease in the signal-to-noise ratio of the implantable flexible photoelectrode device. For example, the minimum excitation power of the current Micro-LED is about 4.8 µW, the light transmission depth is about 35 µm, the influence range is about 40m * 40 µm. Because the light field between two light emitting units 92 is superimposed, the light intensity at a position where the original light intensity is less than the sensing intensity threshold is increased, so that the neuronal cells that would not be sensed is sensed and produce a response, thereby affecting the desired effect.

In the present disclosure, because the light emitting region N is blocked by the first light-shielding layer 104, so that the light emitting region is reduced, the above-mentioned overlap between adjacent light emitting units can be avoided or eliminated, thereby increasing the signal-to-noise ratio of the implantable flexible photoelectrode device.

For example, the first light-shielding layer 104 includes a first light-transmitting part configured to transmit the light beam L therethrough to exit in a direction away from the light emitting region N. In some embodiments, the first light-transmitting part may be a via hole or may be a light-transmitting portion made from a light-transmitting material. When the first light-transmitting part is the via hole, it can reduce the cost of the material and simplify the manufacturing process, thus it is a preferred example. Embodiments of the present invention are described by taking the first light-transmitting part being the via hole as an example.

In embodiments of the present invention, the thickness of the first light-shielding layer 104 may vary according to the light emitting power of the light emitting unit. In the case where the light emitting power is larger, the thickness of the first light-shielding layer 104 is greater, so as to ensure that the light beam can exit from the first light-transmitting part. In some embodiments, the thickness of the first light-blocking layer 104 is 5-10µm.

In embodiments of the present invention, the first light-shielding layer 104 includes a light-blocking material, and the light-blocking material is an insulation material. Preferably, the light-blocking material is a non-biologically toxic insulating material, for example, it can be selected from the group consisting of polyvinylidene fluoride, alkyl sulfonate, ethylene triamine, and ethylene glycol. It can be contemplated that other materials suitable for use, such as black matrix (BM) materials and the like, may also be used in embodiments of the present invention.

In some embodiments, the first light-shielding layer 104 may be a single-layer or multi-layer structure.

For example, in the case that the first light-shielding layer 104 has a single-layer structure, the amount of the material used as the first light-shielding layer 104 can be reduced, and the difficulty of the manufacturing process can be reduced. In this case, the first light-shielding layer 104 may be made from the above-described light-blocking material.

For example, in the case that the first light-blocking layer 104 has a multi-layer structure, it may have several different functions. For example, the first light shielding layer 104 includes a plurality of insulating sub-layers, the plurality of insulating sub-layers include a first insulating sub-layer and a second insulating sub-layer laminated together. The first insulating sub-layer is close to the light emitting assembly and configured as an encapsulation layer of the light emitting assembly, and the second insulating sub-layer is located on a side of the first insulating sub-layer away from the light emitting assembly and configured to shield the light. Thus, in the case that the first light-shielding layer 104 has the multi-layer structure, it has not only the light-shielding function but also the sealing function for the light emitting assembly, thereby avoiding the entry of moisture. In some embodiments, the first insulating sub-layer is made from an organic material, and the second insulating sub-layer is made from the above-mentioned light blocking material.

As shown in FIGS. 6 and 7, the first light-transmitting part includes a first via hole 121 provided in the first light-shielding layer 104, an orthographic projection of the first via hole 121 on the first flexible support layer 101 is located within an orthographic projection of the light emitting region N on the first flexible support layer 101. That is, the area of the first via hole 121 is smaller than the area of the light emitting region N, which is more beneficial to generating approximately collimated irradiation light.

The planar shape of the first via hole 121 includes at least one of a square, a circle, an ellipse, a rectangle, and a parallelogram. It can be contemplated that the planar shape of the first via hole 121 may also be other shapes, and embodiments of the present invention are not limited thereto.

The size of the first via hole 121 is 0.1-99% of the size of the light emitting region N. Further, for example, the size of the first via hole 121 is 0.1% to 56% of the size of the light emitting region N, further, the size of the first via hole 121 is 5% to 50% of the size of the light emitting region N, still further, the size of the first via hole 121 is 15% to 35% of the size of the light emitting region N. If the above-mentioned size are too large or too small, the manufacturing difficulty may be increased.

The area of the light emitting region N is 400-900 µm², and the area of the first via 121 is 1-225 µm². The light emitting region can be made relatively large to guarantee the light emitting quality. Further, in order to further guarantee the light-emitting quality and light-emitting efficiency, the light emitting region N has an area of 500-800 µm², and the first via hole 121 has an area of 20-200 µm², further 20-50 µm², so that it is possible to prevent a large amount of light from being blocked by the first light-shielding layer and converted into thermal energy to affect the reaction of the target object to the electrical stimulation. For example, in the case that the area of the light emitting region N of each Micro-LED is 22µm*22µm, it is preferable that the area of the first via 121 is 5µm*5µm.

As shown in FIG. 6, a plurality of the light emitting units 110 are provided, and the plurality of light emitting units 110 have a plurality of light emitting regions N. A plurality of first via holes 121 are provided, and the plurality of first via holes 121 are disposed in one-to-one correspondence with the plurality of light emitting regions N. With the above arrangement, the plurality of first via holes 121 is arranged more regularly, which is beneficial to reducing the manufacturing difficulty and is suitable for use in the case of irradiating a plurality of target objects which are arranged regularly.

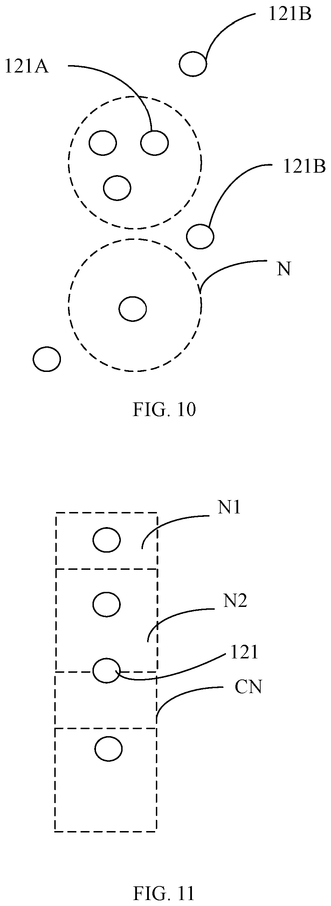

FIG. 10 is a partial plan view of a light emitting region and a first via hole provided by an embodiment of the present invention. For example, FIG. 10 is another variation (using only two light emitting regions as an example) of the light emitting region and the first via hole shown in FIG. 6. As shown in FIG. 10, the first via holes can be freely designed as desired. The various light emitting regions of the Micro-LED may correspond to a different number of the first via holes.

In an actual application scenario, there may be a case that different light irradiating regions are provided with different numbers of target objects, and if the one-to-one regular distribution manner of the first via holes in FIG. 6 is adopted, the requirement for the above application scenario cannot be met. In the present embodiment, by setting the various light emitting regions of the Micro-LED to correspond to the different number of first via holes, different numbers of target objects can be irradiated in the different light irradiating regions, thereby increasing the light irradiation efficiency and making the application scenario of the implantable flexible photoelectrode device more widespread.

Each light emitting region N corresponds to two or more first via holes. In this way, when the same light irradiating region is irradiated, a plurality of light beams L can be generated to irradiate a plurality of target objects respectively in the same light irradiating region, thereby increasing light irradiation efficiency.

As shown in FIG. 10, for example, the orthographic projection of part of the plurality of first via holes 121A on the first flexible support layer 101 is located within the orthographic projection of the light emitting region N on the first flexible support layer 101, and the orthographic projection of another part of the plurality of first via holes 121B on the first flexible support layer 101 is located outside the orthographic projection of the light emitting region N on the first flexible support layer 101. When the plurality of first via holes are arranged in the above-described manner, the plurality of light beams L emitted from the plurality of first via holes have different light intensities. For example, the light intensity of the light beam L emitted from the first via hole 121A is greater than the light intensity of the light beam L emitted from the second via hole 121B. In this way, the requirements for different light intensities to different target objects can be met.

FIG. 11 is a partial plan view of a light emitting region and a first via hole provided by another embodiment of the present invention. For example, FIG. 11 is yet another variation (using only four light emitting regions as an example) of the light emitting region and the first via hole shown in FIG. 6.

As shown in FIG. 11, for example, the plurality of light emitting regions N includes a first light emitting region N1 and a second light emitting region N2. There is no gap between the first light emitting region N1 and the second light emitting region N2, so as to connect the first light emitting region N1 and the second light emitting region N2 with each other. By connecting the first light emitting region N1 and the second light emitting region N2 with each other, a shared light emitting region CN may be formed. In the case that the plurality of first via holes 121 are provided, it is not necessary to position each of the first via holes 121 within the light emitting region corresponding thereto, that is, a certain deviation in position of the first via holes 121 is allowed. With the above arrangement, not only the difficulty of the manufacturing process can be simplified, but also the flexibility on the layout of the first via holes 121 can be increased.

As shown in FIGS. 6 and 7, the implantable flexible photoelectrode device 1 further includes an electrode assembly 103, the electrode assembly 103 is configured to record and transmit a signal generated upon excitation by the light beam. The signal may be generated in the light irradiating region M or in a light non-irradiating region. The electrode assembly 103 has a function to record changes in the electrical signal of the target object (e.g., neuronal cells) under light irradiation or light regulation, and to transmit the electrical signal to the control device.

The electrode assembly 103 and the light emitting assembly 102 may be provided on the same connecting part (e.g., both on the first connecting part 11 as shown in FIG. 6), or may be on different connecting part respectively. In the case that the two are located on different connecting parts, respectively, it can prevent the light beam for irradiating the light irradiating region from irradiate the electrode assembly 103, thereby reducing the interference of the light beam on the electrode recording and increasing the signal-to-noise ratio of the device. In the case that both are located on the same connecting part, the number of connecting parts can be reduced, thereby reducing the number of implanting openings (i.e., the openings on the brain surface for guiding the implantable flexible photoelectrode device) on the brain surface. Thus, a person skilled in the art can set the specific positions of the electrode assembly 103 and the light emitting assembly 102 according to actual needs.

As shown in FIGS. 6 and 7, the electrode assembly 103 is located on the first connecting part 11 and includes a first electrode 111. The first electrode 111 and the light emitting unit 110 are both located on the first side 101A of the first flexible support layer 101; the first electrode 111 and the light emitting unit 110 are spaced apart from each other in a direction parallel to a plane where the first flexible support layer 101 is located. That is, the light emitting region N is spaced apart from the first electrode 111, i.e., an orthographic projection of the light emitting region N on the plane where the first flexible support layer 101 is located does not overlap with an orthographic projection of the first electrode 111 on the plane where the first flexible support layer 101 is located. Thus, the interference of the light beam L on the first electrode 111 can be further reduced, and the signal-to-noise ratio of the device can be increased.

As illustrated in FIG. 7, the first light-shielding layer 104 further includes a second light-transmitting part, the position of the second light-transmitting part is arranged to correspond to the first electrode 111 so as to expose at least part of the first electrode 111. The second light-transmitting part is configured to transmit external ambient light. The second light-transmitting part may be a via hole or may be a light-transmitting portion made from a light-transmitting material. In the case that the second light-transmitting part is a via hole, it can reduce the cost of the material and simplify the manufacturing process. The present disclosure are described by taking the second light-transmitting part being the via hole as an example.

As illustrated in FIGS. 6 and 7, f the second light-transmitting part is a second via hole 122 located in the first light-shielding layer 104, and the second via hole 122 exposes part of the first electrode 111. Compared with the case where the first light shielding layer 104 covers the first electrode 111, by exposing part of the first electrode 111, the precision and accuracy of the first electrode 111 in recording changes of electrical signals can be increased. Further, in the case that the second via hole 122 is arranged to expose the entire first electrode 111, the detection precision and accuracy of the first electrode 111 can be further increased.

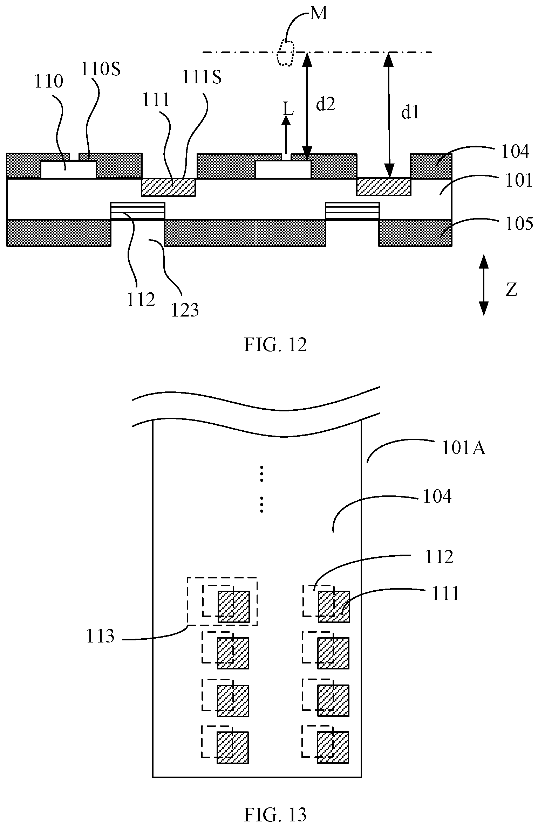

FIG. 12 is a cross-sectional view of a first electrode and a light emitting unit provided by the present disclosure. FIG. 12 is another variation of the first connecting part of FIG. 6.

As shown in FIG. 12, a surface 111S of the first electrode 111 close to the light irradiating region is not coplanar with the light emitting surface 110S of the light emitting unit 110. Thus, it further reduces the interference of the light beam emitted from the light emitting unit 110 on the first electrode 111 and increase the accuracy of the first electrode 111 in recording changes of the electric signals. Further, a distance d1 from the surface 111S of the first electrode 111 to the light irradiating region M is greater than a distance d2 from the light emitting surface 110S of the light emitting unit 110 to the light irradiating region M, that is, the surface 111S is farther from the light irradiating region M than the light emitting surface 110S. As can be seen from FIG. 12, the light beam L emitted from the light emitting surface 110S is directed toward the light irradiating region M; in the case that the surface 111S is farther from the light irradiating region than the light emitting surface 110S, the photoelectric effect generated by the light beam L impinging on the first electrode 111 (since the first electrode 111 is on the back side of the light emitting unit 110) can be avoided, thereby avoid affecting the accuracy of detection signals of the first electrode 111.

Returning to FIG. 7, the electrode assembly 103 further includes a second electrode 112; the second electrode 112 is located on a side of the first electrode 111 away from the light irradiating region M and is insulated from the first electrode 111. While the signals of neuronal cells in the brain are acquired, the signals vary according to the implantation depth. By setting up a double-layer electrode structure as the first electrode 111 and the second electrode 112, the signals acquired by each of the first electrode 111 and the second electrode 112 can be compared and analyzed, which is beneficial to increasing the detection accuracy.

In the present disclosure, the first electrode 111 and the second electrode 112 are insulated from each other by the first flexible support layer 101. The first flexible support layer 101 is made from a flexible insulating material, such as parylene C, polyethylene terephthalate (PETE), or polydimethylsiloxane (PDMS). The first electrode 111 and the second electrode 112 may be made from an electrically conductive material. The electrically conductive material is at least one of a non-metallic material and a metallic material. The non-metallic material is for example poly (3, 4-ethylenedioxythiophene) or polystyrene sulfonate (PEDOT: PSS). The metal material is for example at least one of gold, silver, copper.

As shown in FIG. 7, the second electrode 112 and the light emitting unit 110 are spaced apart from each other in a direction parallel to a plane where the first flexible support layer 101 is located. That is, a spacing is left between the light emitting region N and the second electrode 112, i.e., an orthographic projection of the light emitting region N on the plane where the first flexible support layer 101 is located does not overlap with an orthographic projection of the second electrode 112 on the plane where the first flexible support layer 101 is located. Thus, the interference of the light beam L on the second electrode 112 can be further reduced and the signal-to-noise ratio of the device can be increased.

As shown in FIG. 7, the first connecting part 11 further includes a second light-shielding layer 105 located on a side of the first flexible support layer 101 away from the light emitting assembly 102, i.e., located on the second side 101B of the first flexible support layer 101. By providing the second light-shielding layer 105 on the second side 101B, the light beam L emitted by the light emitting unit 110 can be prevented from exiting from the second side 101B of the first flexible support layer 101, thereby avoiding the occurrence of light leakage.

As illustrated in FIG. 7, the second light-shielding layer 105 includes a third light-transmitting part, the position of the third light-transmitting part is arranged to correspond to the second electrode 112 so as to expose at least part of the second electrode 112. The third light-transmitting part is configured to transmit external ambient light. In some embodiments, the third light-transmitting part may be a via hole or may be a light-transmitting portion made from a light-transmitting material. In the case that the third light-transmitting part is a via hole, it can reduce the cost of the material and simplify the manufacturing process, thus it is a preferred example. The present disclosure are described by taking the third light-transmitting part being the via hole as an example.

As shown in FIG. 7, the third light-transmitting part is a third via hole 123 in the second light-shielding layer 105, and the third via hole 123 exposes part of the second electrode 112. Compared with the case where the second light-shielding layer 105 covers the second electrode 112, by exposing part of the second electrode 112, the precision and accuracy of the second electrode 112 in recording changes of electrical signals can be increased. Further, in the case that the second via hole 122 is arranged to expose the entire second electrode 112, the detection precision and accuracy of the second electrode 112 can be further increased.

As shown in FIG. 12, the first electrode 111 and the second electrode 112 are both located inside the first flexible support layer 101, and the first electrode 111 and the second electrode 112 are insulated from each other by the first flexible support layer 101. In FIG. 12, the third via hole 123 in the second light-shielding layer 105 exposes the entire second electrode 112, so that the detection accuracy of the second electrode 112 can be increased.

In the present disclosure, the number of the first electrode 111 and the number of the second electrode 112 may be the same or may be different. In the case that the numbers of the first electrode 111 and the second electrode 112 are the same, the design difficulty can be reduced and the manufacturing process can be simplified, thus it is a preferred example.

FIG. 13 is a structural schematic diagram of a first electrode and a second electrode provided by the present disclosure. FIG. 13 is a structural schematic diagram of the first electrode 111 and the second electrode 112 of FIG. 7. As shown in FIG. 13, the plurality of first electrodes 111 and the plurality of second electrodes 112 are the same in number, and are disposed in one-to-one correspondence to constitute a plurality of electrode groups 113.

With reference to FIGS. 6, 7, and 13, each of the light emitting units 110 is disposed in one-to-one correspondence with one of the electrode groups 113, so that when the light beam emitted from the light emitting unit 110 irradiates the target object in the light irradiating region, the electric signal generated by the target object can be simultaneously recorded by a pair of the first electrode 111 and the second electrode 112 in the electrode group 113.

As shown in FIG. 13, the first electrode 111 and the second electrode 112 overlap with each other in the thickness direction perpendicular to the first flexible support layer, which is beneficial to detecting the same target object by using both electrodes. Further, in the case that the first electrode 111 and the second electrode 112 completely overlap with each other, both electrodes can detect the same target object at the same position, thereby further increasing the detection accuracy.

In the present disclosure, the arrangement of the plurality of first electrodes 111 and the arrangement of the plurality of second electrodes 112 may be the same or different. In the case that both of them are arranged in the same manner, the design difficulty can be reduced and the manufacturing process can be simplified, thus it is a preferred example. As shown in FIG. 13, the plurality of first electrodes 111 and the plurality of second electrodes 112 are arranged in an array.

In the present disclosure, the electrode assembly 103 further includes a first electrode line and a second electrode line (not shown). The first electrode 111 is connected with the control device inside the housing 2 through the first electrode line, and the second electrode 112 is connected with the control device inside the housing 2 through the second electrode line. By providing the first electrode line and the second electrode line, the electrical signals recorded by the first electrode 111 and the second electrode 112 can be transmitted to the control device, respectively.

For example, the first electrode line and the second electrode line are both located in the first connecting part. The first electrode line and the second electrode line are for example made from an electrically conductive material.

Returning to FIG. 4, the implantable flexible photoelectrode device 1 further includes a fixing assembly 13, the fixing assembly 13 is located on the main body 10 and configured to fix the main body 10 in place. When the implantable flexible photoelectrode device 1 is implanted into the brain through an implanting opening of the brain surface, in order to keep the relative position of the main body 10 unchanged, the main body 10 can be fixed in the vicinity of the implanting opening of the brain surface by using the fixing assembly 13.

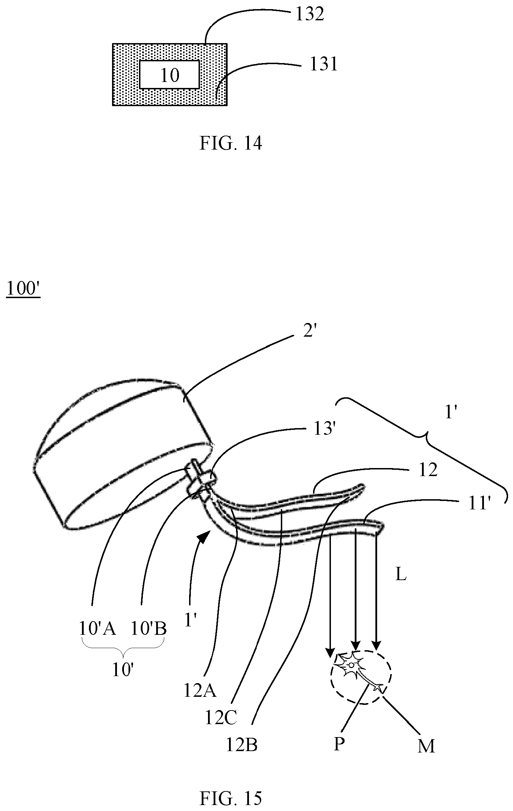

The present disclosure do not limit the specific implementation of the fixing assembly 13, as long as the main body 10 can be fixed in place by using the fixing assembly 13. FIG. 14 is a schematic cross-sectional view of the fixing assembly 13 of FIG. 4. As shown in FIG. 14, the fixing assembly 13 includes an adhesive 131 and a storage container 132 for storing the adhesive 131. The main body 10 has a circumferential direction perpendicular to its extension direction (for example, the extension direction is a direction from an inner side to a outer side of the paper of FIG. 14), and the storage container 132 is disposed along the circumferential direction of the main body 10, so that the adhesive 131 within the storage container 132 can also be disposed along the circumferential direction of the main body 10 and fix the main body 10 to the implanting opening of the brain surface.

In conjunction with FIGS. 4 and 14, the storage container 132 may be located on the first end 10A of the main body 10. By providing the storage container 132 on the first end 10A of the main body 10, the storage container 132 can be located on a side of the main body 10 far away from the light emitting assembly 102, thereby preventing the storage container 132 from affecting the light beam emitted by the light emitting assembly 102.

If there is no adhesive to fix the position of the main body 10, the position of the light emitting assembly 102 may shift, resulting in a shift in the light irradiating region, which in turn affects the light stimulation signal. In the present embodiment, by fixing the light emitting assembly 102 in such a manner that the adhesive 131 is disposed at a position far away from the light emitting assembly 102 and the first end 10A of the main body 10 is fixed by the adhesive 131, the influence on the light stimulation signal when the light emitting assembly 102 is shifted can be avoided.

The storage container 132 shown in FIG. 4 is merely illustrative; in other embodiments, the storage container 132 may be located in the first region 11A of the first connecting part 11, which is also far away from the lighting emitting assembly 102, so as not to affect the normal working of the lighting emitting assembly 102.

In some embodiments, the storage container 132 is configured to automatically release the adhesive 131 when the implantable flexible photoelectrode device 100 is fixed in place. For example, the storage container 132 automatically releases the adhesive 131 when the implantable flexible photoelectrode device 100 is implanted at a position below the cranial bones of the brain. Compared to manually release the adhesive, the operation time can be reduced and the implantation speed can be increased.

In embodiments of the present invention, the storage container 132 is for example a sealing pouch made from a polymeric material, such as polyvinylchloride (PVC) or polycarbonate (PC). Similar to a hand-tearing opening of the food packages, the sealing package can be manually torn. According to actual needs, one or more implantable flexible photoelectrode devices 1 may be provided with the sealing pouch.

In embodiments of the present invention, the adhesive 131 is for example a biological gel, and the biological gel is a medical adhesive gel which has the functions of hemostasis, adhesion, breathability, antibiosis, and the like. The biological gel includes a readily degradable biological gel or a non-readily degradable biological gel. For example, the readily degradable biological gel includes sodium alginate gel or geltin methacryloyl (GELMA). The non-readily degradable biological gel includes polyvinyl alcohol (PVA) hydrogels, polyacrylamide-alginic acid (PAAM) hydrogels, or polyacrylic acid (PAA) hydrogels.

Returning to FIGS. 4 and 5, the brain-computer interface apparatus 100 includes a control device 30. The control device 30 is located in the housing 2 and connected with the implantable flexible photoelectrode device 1. The control device 30 is configured for signal transmission with the implantable flexible photoelectrode device 1. Because the control device 30 is located in the housing 2 and the control device 30 is connected with the implantable flexible photoelectrode device 1, when the control device 30 and the housing 2 move together, the main body of the implantable flexible photoelectrode device 1 also moves together. Therefore, the brain-computer interface apparatus according to embodiments of the present invention is highly integrated and portable, and can be used to do real-time monitoring of the target object anytime and anywhere.

As illustrated in FIG. 5, for example, the control device 30 includes a central control module 31, a drive module 32, a detection module 33, and a power supply module 34.

For example, one end of the central control module 31 is connected to the drive module 32 and configured to send a drive signal to the drive module 32, and the other end thereof is connected to the detection module 33 and configured to receive a detection feedback signal from the detection module 33. In some embodiments, the central control module 31 is, for example, an integrated circuit.

For example, one end of the driving module 32 is connected with the central control module 31, and the other end is connected with the light emitting assembly 102 of the implantable flexible photoelectrode device 1. The drive module 32 is configured to receive the drive signal from the central control module 31 and transmit the drive signal to the light emitting assembly 102. The drive signal is, for example, a voltage for driving the light emitting assembly 102 to emit light.

For example, one end of the detection module 33 is connected to the central control module 31 and the other end is connected to the electrode assembly 103 of the implantable flexible photoelectrode device 1. The detection module 33 is configured to receive a signal (for example a detection feedback signal) from the electrode assembly 103 and transmit the signal to the central control module 31.

Further, for example, the central control module 31 includes a control circuit 311 and a communication circuit 312 connected with the control circuit 311. One end of the communication circuit 312 is connected to the drive module 32 and the other end is connected to the detection module 33. On the one hand, the control circuit 311 generates the drive signal and transmits it to the drive module 32 via the communication circuit 312, and on the other hand, the communication circuit 312 transmits the detection feedback signal provided by the detection module 33 to the control circuit 311.

In some embodiments, in the case that the brain-computer interface apparatus includes a plurality of implantable flexible photoelectrode devices, the plurality of central control modules 31 in the plurality of photoelectrode devices may communicate with each other through respective communication circuits 312, which is beneficial to comparing optical or electrical signals between various flexible photoelectrode devices, so as to enhance the regulation capability.

For example, the power supply module 34 is connected to the central control module 31, the drive module 32, and the detection module 33 respectively to supply power. Compared with the case that the power module 34 is arranged outside the housing 2, arranging the power module 34 inside the housing 2 may further increase the integration of the brain-computer interface apparatus 100.

In some embodiments, the power module 34 is for example a lithium battery or other rechargeable batteries. For example, the operating power of a single Micro-LED is as low as 4.8µW, its power-supply voltage is in the range of 3-6V, and its operating current is in the microampere level, thus only a small-capacity lithium battery is required.

In the previous embodiments, the light emitting assembly 102 and the electrode assembly 103 are both located on the first connecting part 11, so that the driving module 32 and the detection module 33 are both connected to the central control module 31, and the three modules may be located on the same chip. However, when the light emitting assembly 102 and the electrode assembly 103 are located on different connecting parts, the driving module 32 and the detection module 33 may be separately provided. For example, the driving module 32 and a first central control module connected with the driving module 32 are located on one chip, while the detection module 33 and a second central control module connected with the detection module 33 are located on another chip. In the case that the driving module 32 and the detection module 33 are provided on different chips, signal crosstalk between the drive module 32 and the detection module 33 can be reduced. In the case that the driving module 32 and the detection module 33 are provided on the same chip, the integrated degree of the brain-computer interface apparatus can be increased.

In the previous embodiments, both the light emitting assembly 102 and the electrode assembly 103 are located on the first connecting part 11, so that the integrated degree of the implantable flexible photoelectrode device can be increased, which is beneficial to the miniaturization of the device. However, the light emitting assembly 102 and the electrode assembly 103 may also be provided separately. In the first situation, the light emitting assembly 102 and the electrode assembly 103 are provided on two connecting parts of the same implantable flexible photoelectrode device, respectively. In the second situation, the light emitting assembly 102 and the electrode assembly 103 are provided on different connecting parts of different implantable flexible photoelectrode devices, respectively. In order to simplify the description, the description will be made below by taking the first situation as an example.

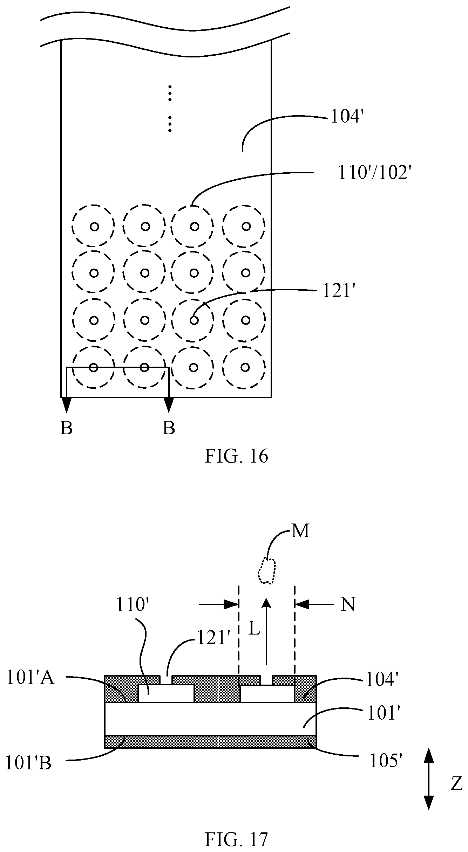

FIG. 15 is a block diagram of a brain-computer interface apparatus provided by another embodiment of the present invention. FIG. 16 a partially enlarged schematic diagram of a first connecting part of FIG. 15. FIG. 17 is a schematic cross-sectional view taken along line BB of FIG. 16.

As shown in FIG. 15, another embodiment of the present invention provides a brain-computer interface apparatus 100', which includes: one implantable flexible photoelectrode device 1', a housing 2', and a control device (not shown) located in the housing 2'. Unlike the brain-computer interface apparatus 100 of FIG. 4, the brain-computer interface apparatus 100' of FIG. 15 includes two connecting parts.

For example, the implantable flexible photoelectrode device 1' includes a main body 10', and a first connecting part 11' and a second connecting part 12 which are connected to the main body 10'. The main body 10' includes a first end 10'A and a second end 10'B oppositely arranged along an extension direction of the main body10'; the first end 10'A is connected with the housing 2 and the second end 10'B is connected with both the first connecting part 11' and the second connecting part 12.

As shown in FIG. 17, for example, the first connecting part 11' includes a first flexible support layer 101', a light emitting assembly 102', and a first light shielding layer 104'. For example, the light emitting assembly 102' is located on the first flexible support layer 101 and is configured to provide a light beam L to a light irradiating region M (shown with reference to FIG. 1).

In some embodiments, as shown in FIGS. 15-17, the first flexible support layer 101' includes a first side 101'A and a second side 101'B that are oppositely arranged in a thickness direction perpendicular to the first flexible support layer 101' (e.g., the Z direction in the figure), and the light emitting assembly 102'is located on at least one of the first side 101'A and the second side 101'B.

As shown in FIG. 15, for example, when the target object P is located at the first side 101'A of the first flexible support layer 101' (i.e., the lower side of the first connecting part 11' in FIG. 15), the light emitting assembly 102' is arranged at the first side 101'A, such that the light beam L emitted by the light emitting assembly 102' can irradiate the target object P in the light irradiating region M. When there are a plurality of target objects P distributed on both the first side 101'A and the second side 101'B of the first flexible support layer 101', each of the first side 101'A and the second side 101'B of the first flexible support layer 101' are provided with the light emitting assembly 102', so that the plurality of target objects P can be irradiated. Thus, by providing the light emitting assembly 102'on at least one of the first side 101'A and the second side 101'B, the flexibility of the implantable flexible photoelectrode device 1' in different application scenarios can be increased and various light irradiating requirements can be met. Embodiments of the present invention are described with the light emitting assembly 102' being located on only the first side 101'A.

As shown in FIGS. 16 and 17, for example, the light emitting assembly 102' includes light emitting units 110' each having a light emitting region N and configured to emit a light beam L from the light emitting region N. Embodiments of the present invention do not particularly limit the categories of the light emitting unit 110'. In some embodiments, the light emitting unit 110' may adopt a light emitting diode device, such as a micro light emitting diode (Micro-LED) device or a quantum-dot light emitting diode (QLED) device. Micro-LEDs are preferred over QLEDs because of their high reliability and long lifetime. Here, the specific structure and arrangement manner of the light emitting unit 110' can be referred to the related description in the previous embodiment, and will not be described in detail here.

In some embodiments, at least part region of the first connecting part 11' is provided with the light emitting assembly 102'. For example, the first connecting part 11', along an extension direction of the first connecting part 11', includes: a first region close to the main body 10', a second region away from the main body 10', and a third region located between the first region and the second region. Here, the arrangement manner of the first region, the second region, and the third region of the first connecting part 11' may refer to the arrangement manner of the first region 11A, the second region 11B, and the third region 11C of the first connecting part 11 shown in FIG. 4. The light emitting assembly 102' is located in at least one of the first region 11A, the second region 11B, and the third region 11C. In this way, the position of the light emitting assembly 102' can be flexibly set according to actual needs. Embodiments of the present invention are described by taking the light emitting assembly 102' being located in the first region 11A, the second region 11B, and the third region 11C as an example.

As shown in FIG. 17, for example, the first light-shielding layer 104' is located on a side of the light emitting unit 110' away from the first flexible support layer 101' , and the first light-shielding layer 104' is configured to cover part of the light emitting region N, so that the light beam L is partially blocked by the first light-shielding layer 104'.

In the present embodiment, by providing the first light shielding layer 104' on the light emitting unit 110', the light beam L emitted from the light emitting unit 104' is partially blocked by the first light shielding layer 104', the divergence angle of the light beam L is reduced, and the effect of collimation can be almost achieved, and therefore, the light beam L is more easily concentrated on specific neuronal cells, thereby solving the problem of diffusion of the light path and increasing the light modulation accuracy.

For example, the first light-shielding layer 104' includes a first light-transmitting part configured to transmit the light beam L therethrough to exit in a direction away from the light emitting region N. In some embodiments, the first light-transmitting part may be a via hole or may be a light-transmitting portion made from a light-transmitting material. When the first light-transmitting part is a via hole, it can reduce the cost of the material and simplify the manufacturing process, thus it is a preferred example.

As shown in FIGS. 16 and 17, for example, the first light-transmitting part includes a first via hole 121' provided in the first light-shielding layer 104', an orthographic projection of the first via hole 121' on the first flexible support layer 101' is located within an orthographic projection of the light emitting region N on the first flexible support layer 101'. That is, the area of the first via hole 121' is smaller than the area of the light emitting region N, which is more beneficial to generating approximately collimated irradiation light.

In some embodiments, the light emitting unit further include a first metal pad and a second metal pad, and the light emitting assembly 102' further includes a first power supply line and a second power supply line. The first power supply line is connected with the first metal pad, and the second power supply line is connected with the second metal pad. Here, the specific structures and arrangement manners of the first power supply line, the second power supply line, the first metal pad, and the second metal pad can be referred to the relevant descriptions in the previous embodiments, and will not be described in detail here.

In the present embodiment, the specific structure and arrangement manners of the first flexible support layer 101' , the first light-shielding layer 104', the light emitting assembly 102', the light emitting unit 110', the light emitting region N, and the first via hole 121' can be referred to the relevant description in the previous embodiment, which will not be described in detail here.

As shown in FIG. 16, a plurality of the light emitting units 110' are provided, and the plurality of light emitting units 110' have a plurality of light emitting regions N. A plurality of the first via holes 121' are provided, and the plurality of first via holes 121' are disposed in one-to-one correspondence with the plurality of light emitting regions N. With the above arrangement, the plurality of first via holes 121' are arranged more regularly, which is beneficial to reducing the manufacturing difficulty and is suitable for use in the case of irradiating a plurality of target objects which are arranged regularly.

As shown in FIG. 17, for example, the first connecting part 11 further includes a second light-shielding layer 105', which is located on a side of the first flexible support layer 101' away from the light emitting assembly 102, i.e. on a second side 101'B of the first flexible support layer 101'. By providing the second light-shielding layer 105' on the second side 101'B, the light beam L emitted by the light emitting unit 110' can be prevented from exiting from the second side 101'B of the first flexible support layer 101', thereby avoiding the occurrence of light leakage.

In some embodiments, in the case that the light emitting assembly further includes a light emitting unit disposed on the second side 101'B of the first flexible support layer 101', the second light-shielding layer 105' includes a light-transmitting part configured to transmit the light beam L therethrough. The light-transmitting part is, for example, a via hole, and may be designed with reference to the first via hole 121' .

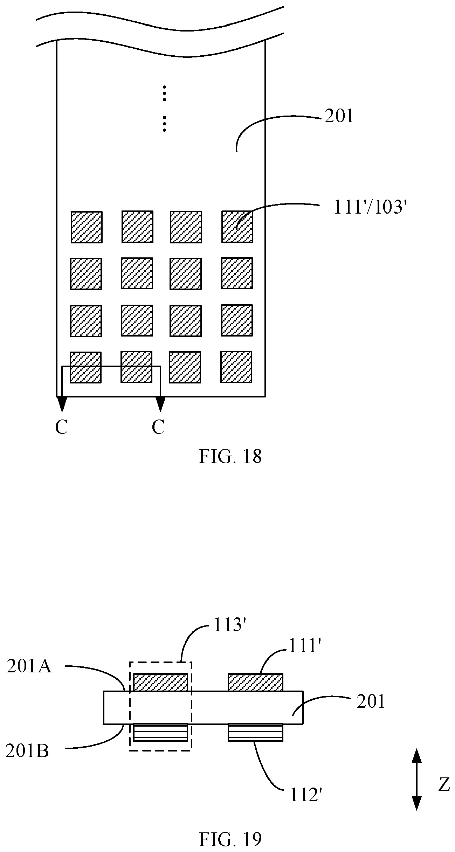

FIG. 18 is a partially enlarged schematic diagram of a second connecting part of FIG. 15. FIG. 19 is a schematic cross-sectional view taken along line CC of FIG. 18.

In embodiments of the present invention, the second connecting part 12 is different from the first connecting part 11'. For example, different components are provided on the second connecting part 12 and the first connecting part 11'. As shown in FIGS. 15 to 19, when the light emitting assembly 102' is located on the first connecting part 11', the electrode assembly 103' is located on at least one of the first connecting part 11' and the second connecting part 12.

As shown in FIGS. 15 to 19, the light emitting assembly 102' is located on the first connecting part 11' and the electrode assembly 103' is located on the second connection 12. By arranging the light emitting assembly 102' and the electrode assembly 103' on different connecting parts, respectively, the light beam L which is emitted from the light emitting assembly 102' and irradiates the electrode assembly 103' can be reduced, thereby reducing interference of the light beam on the electrode recording and increasing the signal-to-noise ratio of the device.

As shown in FIG. 15, the second connecting part 12 and the first connecting part 11' are spaced apart from each other. For example, the second connecting part 12 is located on a side of the first connecting part 11' away from the light irradiating region M, so that the light beam L which is emitted from the light emitting assembly 102' and irradiates the electrode assembly 103' can be further reduced, thereby increasing the signal-to-noise ratio of the device.

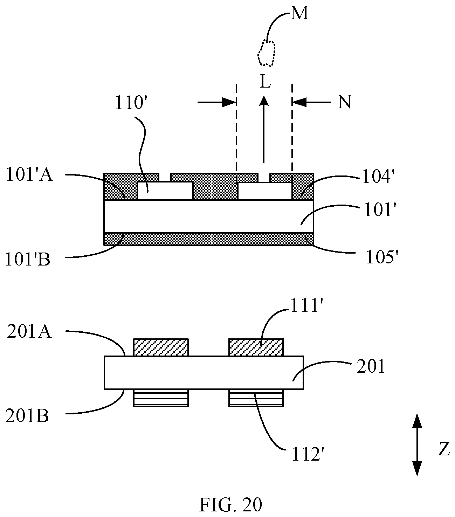

FIG. 20 is a partial cross-sectional view of a first connecting part and a second connecting part provided by an embodiment of the present invention. For example, FIG. 20 is a schematic partial cross-sectional view of the first connecting part 11' and the second connection 12 of FIG. 16.

As shown in FIG. 20, the first connecting part 11' is closer to the light irradiating region M than the second connecting part 12, i.e. the second connecting part 12 is located on a side of the first connecting part 11' away from the light irradiating region M. Thus, the light beam L emitted from the light emitting unit 110' on the first connecting part 11' can be prevented from being emitted to the electrode assembly 103' on the second connecting part 12.

As shown in FIGS. 19 and 20, for example, the second connection 12 includes a second flexible support layer 201. The second flexible support layer 201 includes a first side 201A and a second side 201B that are oppositely disposed in a thickness direction perpendicular to the second flexible support layer 201 (e.g., the Z direction shown in the figure). For example, the first side 201A is close to the light irradiating region M and the second side 201 B is away from the light irradiating region.

For example, the electrode assembly 103' includes a first electrode 111'. The first electrode 111' is located on the first side 201A of the second flexible support layer 201. By arranging the first electrode 111' on the first side 201A of the second flexible support layer 201, the first electrode 111' is located on a side of the first connecting part 11' away from the light irradiating region M, the light beam L can be prevented from being emitted to the first electrode 111'.

As shown in FIGS. 19 and 20, for example, the electrode assembly 103' further includes a second electrode 112', the second electrode 112' is located on the second side 201B of the second flexible support layer 201 and is insulated from the first electrode 111. In some embodiments, while the signals of neuronal cells in the brain are acquired, the signals vary according to the implantation depth. By setting up a double-layer electrode structure as the first electrode 111' and the second electrode 112', the signals acquired by each of the first electrode 111' and the second electrode 112' can be compared and analyzed, which is beneficial to increasing the detection accuracy.

In embodiments of the present invention, the first electrode 111' and the second electrode 112' are insulated from each other by the second flexible support layer 201' . For example, the second flexible support layer 201' is made from a flexible insulating material, such as parylene C, polyethylene terephthalate (PETE), or polydimethylsiloxane (PDMS). The first electrode 111' and the second electrode 112' may be made from an electrically conductive material. For example, the electrically conductive material is at least one of a non-metallic material and a metallic material. The non-metallic material is for example poly (3, 4-ethylenedioxythiophene) or polystyrene sulfonate (PEDOT: PSS). The metal material is for example at least one of gold, silver, copper.

In embodiments of the present invention, the number of the first electrode 111' and the number of the second electrode 112' may be the same or may be different. In the case that the numbers of the first electrode 111' and the second electrode 112' are the same, the design difficulty can be reduced and the manufacturing process can be simplified, thus it is a preferred example.

For example, the first electrode 111' and the second electrode 112' overlap with each other in the thickness direction perpendicular to the first flexible support layer, which is beneficial to detecting the same target object by using both electrodes. Further, in the case that the first electrode 111' and the second electrode 112' completely overlap with each other, both electrodes can detect the same target object at the same position, thereby further increasing the detection accuracy.

As shown in FIG. 16, a plurality of the light emitting units 110' are provided, and the plurality of light emitting units 110' have a plurality of light emitting regions N. A plurality of the first via holes 121' are provided, and the plurality of first via holes 121' are disposed in one-to-one correspondence with the plurality of light emitting regions N. With the above arrangement, the plurality of first via holes 121' are arranged more regularly, which is beneficial to reducing the manufacturing difficulty and is suitable for use in the case of irradiating a plurality of target objects which are arranged regularly.

In some embodiments, the first and second electrodes may be the same or different in number. As shown in FIGS. 18 and 19, a plurality of the first electrodes 111' are provided, a plurality of the second electrodes 112' are provided, and the plurality of first electrodes 111' are the same in number as the plurality of second electrodes 112'. The plurality of first electrodes 111' are disposed in one-to-one correspondence with the plurality of the second electrodes 112' so as to constitute a plurality of electrode groups 113'.

With reference to FIGS. 16 and 18, each of the light emitting units 110' is disposed in one-to-one correspondence with one of the electrode groups 113', so that when the light beam emitted from the light emitting unit 110' irradiates the target object in the light irradiating region, the electric signal generated by the target object can be simultaneously recorded by a pair of the first electrode 111' and the second electrode 112' in the electrode group 113', thus facilitating the subsequent comparison and analysis of the recorded signals.

In embodiments of the present invention, the arrangement of the plurality of first electrodes 111' and the arrangement of the plurality of second electrodes 112' may be the same or different. In the case that both of them are arranged in the same manner, the design difficulty can be reduced and the manufacturing process can be simplified, thus it is a preferred example. For example, as shown in FIG. 18, the plurality of first electrodes 111' and the plurality of second electrodes 112' are arranged in an array.

In embodiments of the present invention, the electrode assembly 103' further includes a first electrode line and a second electrode line (not shown). The first electrode 111' is connected to the control device inside the housing 2' through the first electrode line and the second electrode 112' is connected to the control device inside the housing 2' through the second electrode line. By providing the first electrode line and the second electrode line, the electrical signals recorded by the first electrode 111' and the second electrode 112' can be transmitted to the control device, respectively.

For example, the first electrode line and the second electrode line are both located in the second connecting part 12. The first electrode line and the second electrode line are for example made from an electrically conductive material.

As shown in FIG. 15, the brain-computer interface apparatus 100' includes a control device. The control device is located in the housing 2' and connected with the implantable flexible photoelectrode device 1'. Because the control device is located in the housing 2' and the control device is connected with the implantable flexible photoelectrode device 1', when the control device and the housing 2' move together, the main body of the implantable flexible photoelectrode device 1' also moves together. Therefore, the brain-computer interface apparatus according to embodiments of the present invention is highly integrated and portable, and can be used to do real-time monitoring of the target object anytime and anywhere. The specific structure and arrangement of the control device in the brain-computer interface apparatus 100' can be referred to the previous description in FIG. 4, which is not described in detail here.

Because the housing 2 in FIG. 4 and the housing 2' in FIG. 15 are substantially the same, the following description will be given by taking the housing 2 as an example.

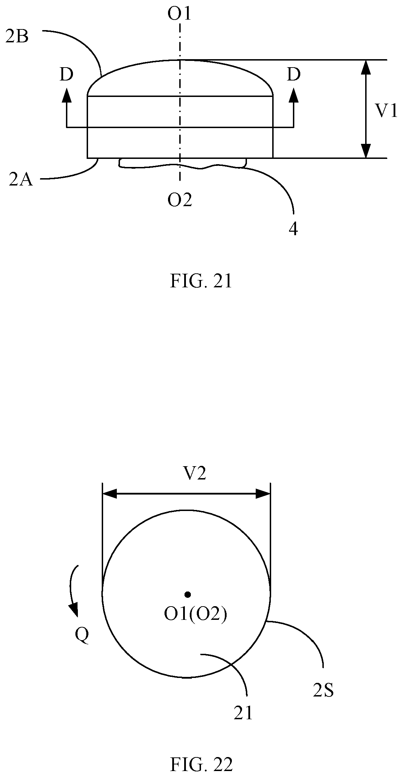

FIG. 21 is a front view of a housing of FIG. 4. FIG. 22 is a cross-sectional view taken along line DD of FIG. 21. As shown in FIGS. 21 and 22, the housing 2 has a longitudinal axis 0102.

For example, the housing 2 has a first size V1 along the longitudinal axis 0102 and a second size V2 perpendicular to the longitudinal axis 0102, and the second size V2 is greater than the first size V1. By setting the second size V2 greater than the first size V1, the height of the housing 2 can be reduced as much as possible on the premise of ensuring that the housing 2 has a large accommodation space (for accommodating the control device). Thus, when the brain-computer interface apparatus 100 is wholly implanted in the brain, the overall implantation depth of brain-computer interface apparatus 100 can be reduced.

In some embodiments, the flexible photoelectrode device 100 is configured to be implanted below cranial bones of the brain, and the housing 2 is configured to be implanted at and/or below cranial bones of the brain. For example, when the housing 2 is implanted at the cranial bone of the brain, an opening may be formed in the cranial bone of the brain, and the housing 2 is disposed in the opening, which is beneficial to fixing the housing 2 and avoiding its shaking. Furthermore, the quality of the signal communication between the brain-computer interface apparatus and the external apparatus (extracorporeal apparatus) and the charging efficiency can be ensured.

In embodiments of the present invention, the number of implantable flexible photoelectrode devices 1 connected to the housing 2 may be one (as shown in FIG. 15) or plural (as shown in FIG. 4). The housing 2 has a circumferential direction Q perpendicular to the longitudinal axis O1O2; in the case that the plurality of implantable flexible photoelectrode devices 1 are provided, the plurality of implantable flexible photoelectrode devices 1 are distributed along the circumferential direction Q, which facilitates the irradiation and electrical signal detection of target objects in different regions of the brain.

In some embodiments, the lengths of the plurality of implantable flexible photoelectrode devices 1 may be the same or different. The person skilled in the art can choose according to the actual needs.

As shown in FIG. 21, for example, the housing 2 includes a first side 2A and a second side 2B oppositely arranged along the longitudinal axis OO, the main body 10 of the implantable flexible photoelectrode device 1 is connected to at least one of the first side 2A and the second side 2B. That is, the implantable flexible photoelectrode device 1 may be provided on a single side of the housing 2 or on both sides. With the above arrangement, the irradiation range and the signal detection range of the implantable flexible photoelectrode device 1 for different regions of the brain can be increased, thereby further increasing the flexibility of the implantable flexible photoelectrode device 1 under different application scenarios. Each of the plurality of implantable flexible photoelectrode devices 1 in FIG. 4 is provided on the first side 2A.

As shown in FIG. 21, for example, the brain-computer interface apparatus 100 further includes a fixing component 4, the fixing component 4 and the main body 10 are located on the first side 2A of the housing 2, and the fixing component 4 is configured to fix the brain-computer interface apparatus 100 in place. For example, in the case that the housing 2 is not implanted in the brain, the fixing component 4 is located between the housing 2 and the surface of the brain and serve; on the one hand, the fixing component 4 plays a role of fixing, on the other hand, it reduces the sensation of foreign objects.

For example, the fixing component 4 includes an adhesive. The adhesive is, for example a biological gel, and the biological gel is a medical adhesive gel which has the functions of hemostasis, adhesion, breathability, antibiosis, and the like. Specific materials for the biological gel are described in detail in the previous examples and will not be described in detail here.

In embodiments of the present invention, a part or an entirety of brain-computer interface apparatus 100 (or brain-computer interface apparatus 100') is configured to be implantable. That is, a part or an entirety of brain-computer interface apparatus 100 may be implanted in the brain. For example, in the case that a part of brain-computer interface apparatus 100 (or brain-computer interface apparatus 100') is implanted in the brain, the implantable flexible optoelectrode device 1 is implanted in the brain and the housing 2 remains outside the brain. In the case that an entirety of the brain-computer interface apparatus 100 (or brain-computer interface apparatus 100') is implanted in the brain, the implantable flexible optoelectrode device 1 is implanted below cranial bones of the brain, and the housing 2 is implanted at and/or below cranial bones of the brain.

The brain-computer interface apparatus provided by embodiments of the present invention may include one or more implantable flexible photoelectrode devices.

The brain-computer interface apparatus 100' of FIG. 15 includes only one implantable flexible photoelectrode device 100', and the first connecting part 11' and the second connection 12 are both connected to the same main body 10'. As another variant, the brain-computer interface apparatus 100' may include at least two implantable flexible photoelectrode devices, and each of the at least two implantable flexible photoelectrode devices includes one main body and one connecting part. For example, one of the at least two implantable flexible photoelectrode devices includes the first connecting part 11' shown in FIG. 15 (i.e. provided with only the light emitting assembly), and another one of the at least two implantable flexible photoelectrode devices includes the second connecting part 12 shown in FIG. 15 (i.e. provided with only the electrode assembly). In this case, the implantable flexible photoelectrode device carrying the light emitting assembly can be used to emitting light signals at one site (i.e., a light irradiating region), and another implantable flexible photoelectrode device carrying the electrode assembly can be used to acquiring signals at the same or a different site (i.e., the light irradiating region or non-light irradiating region). The acquired signals may be used for researching, or further studies of the brain, or may be used in medical devices for treating the abnormal discharges of the epileptic brain.

For example, before the electrode assembly works, the brain tissue is transfected with an inhibitory photosensitive protein; then, a brain abnormal discharge region is identified and brain wave signals at the time of onset are acquired to construct a database; finally, brain signals are detected by the electrode assembly to match with the database. When the brain abnormal discharge is determined, the light assembly can be triggered to emit light to inhibit the brain discharge. By using the above method, the drive program of the implantable flexible photoelectrode device can be trained, so as to accurately stimulate the pathogen when the brain discharges, and minimize the interference on normal cells.

In the case that the brain-computer interface apparatus includes at least two implantable flexible photoelectrode devices, each implantable flexible photoelectrode device may be provided with only the electrode assembly, so that electrical signals can be detected at different parts of the brain. When a subject performs a certain brain activity, such as visual activity, auditory activity, mental activity, etc., the brain signals are accurately detected, which may be used for building up a library of the brain signals or for subsequent simulation and analysis.

In the case that the brain-computer interface apparatus includes at least two implantable flexible photoelectrode devices, each implantable flexible photoelectrode device may be provided with only the light emitting assembly, such that reduction of brain activity or inhibition of brain activity may be achieved. The reduction of brain activity may be a reduction of pain sensation, a reduction of vision, a reduction of touch, a reduction of brain function, and the like; the inhibition of brain activity may be used for specific diseases, such as epilepsy, drug addiction, and the like to inhibit abnormal discharge behavior of the brain.

In the case that the brain-computer interface apparatus includes at least two implantable flexible photoelectrode devices, the arrangement of the electrode assembly and the light emitting assembly is not limited to the cases described in the above-mentioned embodiments. The person skilled in the art can flexibly design according to different needs.

In some embodiments, each of the plurality of implantable flexible photoelectrode devices adopts the configuration of the implantable flexible photoelectrode device 1 shown in FIG. 4. In other embodiments, each of the plurality of implantable flexible photoelectrode devices adopts the configuration of the implantable flexible photoelectrode device 1' shown in FIG. 15. In still other embodiments, some of the plurality of implantable flexible photoelectrode devices adopt the configuration of the implantable flexible photoelectrode device 1 shown in FIG. 4, and the other adopts the implantable flexible photoelectrode device 1' shown in FIG. 15.

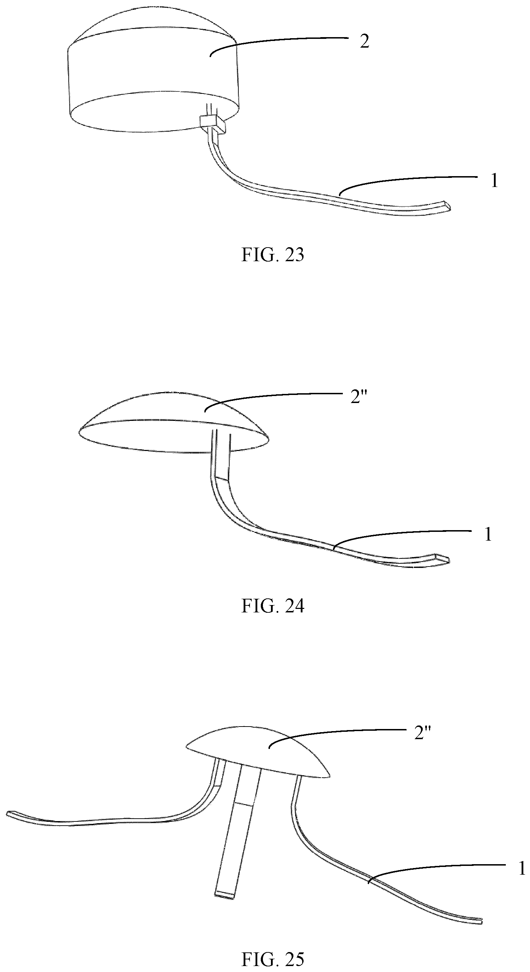

FIG. 23 is a block diagram of a brain-computer interface apparatus provided by the present disclosure. The difference between FIG. 23 and FIG. 4 lies in that the brain-computer interface apparatus of FIG. 23 includes only one implantable flexible photoelectrode device 1.

FIG. 24 is a block diagram of a brain-computer interface apparatus provided by the present disclosure. The difference between FIG. 24 and FIG. 23 lies in that the housing 2" of FIG. 24 is flatter than the housing 2. The first size of the housing 2" is smaller than the first size V1 of the housing 2 in FIG. 21, thereby obtaining a lighter and thinner housing 2".

FIG. 25 is a block diagram of a brain-computer interface apparatus provided by the present disclosure. The difference between FIG. 25 and FIG. 24 lies in that the brain-computer interface apparatus of FIG. 25 includes a plurality of implantable flexible photoelectrode devices 1.

In the present disclosure, the implantable flexible photoelectrode device 1 in FIGS. 23 to 25 may also be replaced with the implantable flexible photoelectrode device 1' of FIG. 15, and the same control device may be simultaneously connected with different categories of implantable flexible photoelectrode devices.

The present disclosure further provides a manufacturing method of a brain-computer interface apparatus, referring to FIG. 4, the manufacturing method includes: forming an implantable flexible photoelectrode device 1. Herein, the implantable flexible photoelectrode device 1 includes: a main body 10 and a first connecting part 11 connected with the main body 10, the first connecting part 11 includes a first flexible support layer 101, a light emitting assembly 102 and a first light shielding layer 104. The light emitting assembly 102 is located on the first flexible support layer 101 and is configured to provide a light beam L to the light irradiating region M. The light emitting assembly 102 includes a light emitting unit 110 having a light emitting region N and configured to emit the light beam L from the light emitting region N. The first light-shielding layer 104 is located on a side of the light emitting unit 110 away from the first flexible support layer 101 and is configured to cover part of the light emitting region N, so as to make the light beam L partially shielded by the first light-shielding layer 104.

In the above-mentioned manufacturing method, by forming the first light-shielding layer on the light emitting unit, the light beam emitted from the light emitting unit is partially blocked by the first light-shielding layer, thereby reducing the area of the light irradiating region. Compared to the case where the light irradiating region in FIG. 3 is relatively larger, t the present disclosure solve the problem of optical path dispersion by reducing the area of the light irradiating region, so that the light beam is more easily to concentrate to a particular neuronal cell, thereby increasing the light modulation accuracy.

Referring to FIGS. 4 and 5, further, the above-mentioned manufacturing method further includes: forming the housing 2 and the control device 30 in the housing 2; herein, the control device 30 is connected with the implantable flexible photoelectrode device 1 and configured for signal transmission with the implantable flexible photoelectrode device 1. The main body 10 of the implantable flexible photoelectrode device 1 is configured to move with the housing 2 and the control device 30.

In the above-mentioned manufacturing method, the specific structures and arrangements of the implantable flexible photoelectrode device 1, the housing 2 and the control device 30 can be referred to the description in the previous disclosure, which will not be described in detail here.

## Claims

1. An implantable flexible photoelectrode device (1, 1'), comprising:
a main body (10, 10');
a first connecting part (11, 11'), connected with the main body (10, 10') and comprising:
a first flexible support layer (101, 101');
a light emitting assembly (102, 102'), on the first flexible support layer (101, 101') and configured to provide a light beam (L) to a light irradiating region (M), the light emitting assembly (102, 102') comprising a light emitting unit (110, 110'), the light emitting unit (110, 110') having a light emitting region (N) and being configured to exit the light beam (L) from the light emitting region (N);
wherein the orthographic projection of the light irradiating region (M) on the first flexible support layer (101, 101') is within the orthographic projection of the light emitting region (N) on the first flexible support layer (101, 101');
wherein the first connecting part (11, 11') further comprises a first light-shielding layer (104, 104'), at a side of the light emitting unit (110, 110') away from the first flexible support layer (101, 101'), the first light-shielding layer (104, 104') being configured to cover part of the light emitting region (N), such that the light beam (L) is partially shielded by the first light-shielding layer (104, 104');
wherein the implantable flexible photoelectrode device (1, 1') further comprises: a second connecting part (12), connected with the main body (10, 10'), the second connecting part (12) being different from the first connecting part (11, 11') and spaced apart from the first connecting part (11, 11'); an electrode assembly (103, 103'), configured to record and transmit a signal generated under excitation of the light beam (L);
**characterized in that**
the electrode assembly (103, 103') is on the second connecting part (12) and comprises: a first electrode (111, 111'), the first electrode (111, 111') being at a side of the first connecting part (11, 11') away from the light irradiating region (M); and a second electrode(112, 112');
wherein the second connecting part (12) comprises a second flexible support layer (201, 201'), the second flexible support layer (201, 201') comprises a first side (201A) close to the light irradiating region (M) and a second side (201B) away from the light irradiating region (M); and the first electrode (111, 111') is at the first side (201A), the second electrode(112, 112') is at the second side (201B), and the second electrode(112, 112') is insulated from the first electrode (111, 111').

2. The implantable flexible photoelectrode device (1, 1') according to claim 1, wherein the first light-shielding layer (104, 104') comprises a first light-transmitting part, the first light-transmitting part is configured to transmit the light beam (L) therethrough to exit in a direction away from the light emitting region (N).

3. The implantable flexible photoelectrode device (1, 1') according to claim 2, wherein the first light-transmitting part comprises a first via hole in the first light-shielding layer (104, 104'), the orthographic projection of the first via hole on the first flexible support layer (101, 101') is located within the orthographic projection of the light emitting region (N) on the first flexible support layer (101, 101').

4. The implantable flexible photoelectrode device (1, 1') according to claim 3,
wherein the first via hole is plural, the orthographic projections of part of a plurality of the first via holes (121, 121') on the first flexible support layer (101, 101') are located within the orthographic projection of the light emitting region (N) on the first flexible support layer (101, 101'), and
the orthographic projections of another part of the plurality of the first via holes (121, 121') on the first flexible support layer (101, 101') are located outside the orthographic projection of the light emitting region (N) on the first flexible support layer (101, 101').

5. The implantable flexible photoelectrode device (1, 1') according to any one of claims 1 to 4,
wherein the light emitting unit (110, 110') is plural, and a plurality of the light emitting units (110, 110') have a plurality of light emitting regions (N);
the first light-shielding layer (104, 104') comprises a plurality of first light-transmitting parts, and the plurality of the first light-transmitting parts are disposed in one-to-one correspondence with the plurality of the light emitting regions (N).

6. The implantable flexible photoelectrode device (1, 1') according to claim 5, wherein the plurality of the light emitting regions (N) are disposed without a gap therebetween, such that the plurality of the light emitting regions (N) are connected to each other.

7. The implantable flexible photoelectrode device (1, 1') of any one of claims 1 to 6, further comprising:
a fixing assembly (13), the fixing assembly (13) being located on the main body (10, 10') and configured to fix the main body (10, 10') in place, wherein the fixing assembly (13) comprises:
an adhesive (131);
a storage container (132), configured to store the adhesive (131);
wherein the main body (10, 10') has a circumferential direction perpendicular to a direction of an extension direction of the main body (10, 10'), the storage container (132) being arranged along the circumferential direction of the main body (10, 10').

8. A brain-computer interface apparatus, comprising the implantable flexible photoelectrode device (1, 1') according to any one of claims 1 to 7.

## Patentansprüche

1. Implantierbare flexible Photoelektrodenvorrichtung (1, 1'), aufweisend:
einen Hauptkörper (10, 10');
ein erstes Verbindungsteil (11, 11'), das mit dem Hauptkörper (10, 10') verbunden ist und aufweist:
eine erste flexible Trägerschicht (101, 101');
eine Lichtemissionsbaugruppe (102, 102'), auf der ersten flexiblen Trägerschicht (101, 101') und dazu konfiguriert, einen Lichtstrahl (L) für einen Lichtbestrahlungsbereich (M) bereitzustellen, wobei die Lichtemissionsbaugruppe (102, 102') eine Lichtemissionseinheit (110, 110') aufweist, wobei die Lichtemissionseinheit (110, 110') einen Lichtemissionsbereich (N) aufweist und dazu konfiguriert ist, den Lichtstrahl (L) aus dem Lichtemissionsbereich (N) austreten zu lassen;
wobei die orthografische Projektion des Lichtbestrahlungsbereichs (M) auf die erste flexible Trägerschicht (101, 101') innerhalb der orthografischen Projektion des Lichtemissionsbereichs (N) auf die erste flexible Trägerschicht (101, 101') liegt;
wobei das erste Verbindungsteil (11, 11') ferner eine erste Lichtabschirmschicht (104, 104') aufweist, an einer Seite der Lichtemissionseinheit (110, 110'), die von der ersten flexiblen Trägerschicht (101, 101') abgewandt ist, wobei die erste Lichtabschirmschicht (104, 104') dazu konfiguriert ist, einen Teil des Lichtemissionsbereichs (N) zu bedecken, sodass der Lichtstrahl (L) teilweise durch die erste Lichtabschirmschicht (104, 104') abgeschirmt wird;
wobei die implantierbare flexible Photoelektrodenvorrichtung (1, 1') ferner aufweist: ein zweites Verbindungsteil (12), das mit dem Hauptkörper (10, 10') verbunden ist, wobei das zweite Verbindungsteil (12) von dem ersten Verbindungsteil (11, 11') verschieden ist und von dem ersten Verbindungsteil (11, 11') beabstandet ist; eine Elektrodenbaugruppe (103, 103'), die dazu konfiguriert ist, ein unter Anregung durch den Lichtstrahl (L) erzeugtes Signal aufzuzeichnen und zu übertragen;
**dadurch gekennzeichnet, dass**
die Elektrodenbaugruppe (103, 103') auf dem zweiten Verbindungsteil (12) ist und aufweist: eine erste Elektrode (111, 111'), wobei die erste Elektrode (111, 111') an einer Seite des ersten Verbindungsteils (11, 11') ist, die von dem Lichtbestrahlungsbereich (M) abgewandt ist; und eine zweite Elektrode (112, 112');
wobei das zweite Verbindungsteil (12) eine zweite flexible Trägerschicht (201, 201') aufweist, wobei die zweite flexible Trägerschicht (201, 201') eine erste Seite (201A) nahe dem Lichtbestrahlungsbereich (M) und eine zweite Seite (201B), die von dem Lichtbestrahlungsbereich (M) abgewandt ist, aufweist; und die erste Elektrode (111, 111') an der ersten Seite (201A) ist, die zweite Elektrode (112, 112') an der zweiten Seite (201B) ist, und die zweite Elektrode (112, 112') gegenüber der ersten Elektrode (111, 111') isoliert ist.

2. Implantierbare flexible Photoelektrodenvorrichtung (1, 1') nach Anspruch 1, wobei die erste Lichtabschirmschicht (104, 104') ein erstes Lichtübertragungsteil aufweist, wobei das erste Lichtübertragungsteil dazu konfiguriert ist, den Lichtstrahl (L) durch sich hindurch zu übertragen, um in einer von dem Lichtemissionsbereich (N) abgewandten Richtung auszutreten.

3. Implantierbare flexible Photoelektrodenvorrichtung (1, 1') nach Anspruch 2, wobei das erste Lichtübertragungsteil ein erstes Durchgangsloch in der ersten Lichtabschirmschicht (104, 104') aufweist, wobei die orthografische Projektion des ersten Durchgangslochs auf die erste flexible Trägerschicht (101, 101') innerhalb der orthografischen Projektion des Lichtemissionsbereichs (N) auf die erste flexible Trägerschicht (101, 101') gelegen ist.

4. Implantierbare flexible Photoelektrodenvorrichtung (1, 1') nach Anspruch 3,
wobei das erste Durchgangsloch in einer Mehrzahl vorgesehen ist, wobei die orthografischen Projektionen eines Teils einer Mehrzahl der ersten Durchgangslöcher (121, 121') auf die erste flexible Trägerschicht (101, 101') innerhalb der orthografischen Projektion des Lichtemissionsbereichs (N) auf die erste flexible Trägerschicht (101, 101') gelegen sind, und
die orthografischen Projektionen eines anderen Teils der Mehrzahl der ersten Durchgangslöcher (121, 121') auf die erste flexible Trägerschicht (101, 101') außerhalb der orthografischen Projektion des Lichtemissionsbereichs (N) auf die erste flexible Trägerschicht (101, 101') gelegen sind.

5. Implantierbare flexible Photoelektrodenvorrichtung (1, 1') nach einem der Ansprüche 1 bis 4,
wobei die Lichtemissionseinheit (110, 110') in einer Mehrzahl vorgesehen ist und eine Mehrzahl der Lichtemissionseinheiten (110, 110') eine Mehrzahl von Lichtemissionsbereichen (N) aufweist;
die erste Lichtabschirmschicht (104, 104') eine Mehrzahl von ersten Lichtübertragungsteilen aufweist und die Mehrzahl der ersten Lichtübertragungsteile in Eins-zu-Eins-Entsprechung zu der Mehrzahl der Lichtemissionsbereiche (N) platziert ist.

6. Implantierbare flexible Photoelektrodenvorrichtung (1, 1') nach Anspruch 5, wobei die Mehrzahl der Lichtemissionsbereiche (N) ohne einen Zwischenraum dazwischen platziert ist, sodass die Mehrzahl der Lichtemissionsbereiche (N) miteinander verbunden ist.

7. Implantierbare flexible Photoelektrodenvorrichtung (1, 1') nach einem der Ansprüche 1 bis 6, ferner aufweisend:
eine Befestigungsbaugruppe (13), wobei die Befestigungsbaugruppe (13) auf dem Hauptkörper (10, 10') gelegen ist und dazu konfiguriert ist, den Hauptkörper (10, 10') an Ort und Stelle zu fixieren, wobei die Befestigungsbaugruppe (13) aufweist:
einen Klebstoff (131);
einen Speicherbehälter (132), der dazu konfiguriert ist, den Klebstoff (131) zu speichern;
wobei der Hauptkörper (10, 10') eine Umfangsrichtung aufweist, die senkrecht zu einer Richtung einer Erstreckungsrichtung des Hauptkörpers (10, 10') ist, wobei der Speicherbehälter (132) entlang der Umfangsrichtung des Hauptkörpers (10, 10') angeordnet ist.

8. Gehirn-Computer-Schnittstellengerät, aufweisend die implantierbare flexible Photoelektrodenvorrichtung (1, 1') nach einem der Ansprüche 1 bis 7.

## Revendications

1. Dispositif à photoélectrode flexible implantable (1, 1'), comprenant :
un corps principal (10, 10') ;
une première partie de connexion (11, 11'), connectée avec le corps principal (10, 10') et comprenant :
une première couche de support flexible (101, 101') ;
un ensemble électroluminescent (102, 102'), sur la première couche de support flexible (101, 101') et configuré pour fournir un faisceau lumineux (L) à une région irradiant de la lumière (M), l'ensemble électroluminescent (102, 102') comprenant une unité électroluminescente (110, 110'), l'unité électroluminescente (110, 110') présentant une région électroluminescente (N) et étant configurée pour faire sortir le faisceau lumineux (L) de la région électroluminescente (N) ;
dans lequel la projection orthographique de la région irradiant de la lumière (M) sur la première couche de support flexible (101, 101') est comprise dans les limites de la projection orthographique de la région électroluminescente (N) sur la première couche de support flexible (101, 101') ;
dans lequel la première partie de connexion (11, 11') comprend en outre une première couche faisant écran à la lumière (104, 104'), sur un côté de l'unité électroluminescente (110, 110') à distance de la première couche de support flexible (101, 101'), la première couche faisant écran à la lumière (104, 104') étant configurée pour couvrir une partie de la région électroluminescente (N) , de telle sorte qu'il est partiellement fait écran au faisceau lumineux (L) par la première couche faisant écran à la lumière (104, 104') ;
dans lequel le dispositif à photoélectrode flexible implantable (1, 1') comprend en outre : une seconde partie de connexion (12), connectée avec le corps principal (10, 10'), la seconde partie de connexion (12) étant différente de la première partie de connexion (11, 11') et espacée de la première partie de connexion (11, 11'), un ensemble d'électrode (103, 103'), configuré pour enregistrer et transmettre un signal généré sous excitation du faisceau lumineux (L) ;
**caractérisé en ce que**
l'ensemble d'électrode (103, 103') se trouve sur la seconde partie de connexion (12) et comprend : une première électrode (111, 111'), la première électrode (111, 111') étant sur un côté de la première partie de connexion (11, 11') à distance de la région irradiant de la lumière (M), et une seconde électrode (112, 112') ;
dans lequel la seconde partie de connexion (12) comprend une seconde couche de support flexible (201, 201'), la seconde couche de support flexible (201, 201'), comprend un premier côté (201A) proche de la région irradiant de la lumière (M) et un second côté (201B) à distance de la région irradiant de la lumière (M), et la première électrode (111, 111') se trouve sur le premier côté (201A), la seconde électrode (112, 112') se trouve sur le second côté (201B) , et la seconde électrode (112, 112') est isolée de la première électrode (111,111').

2. Dispositif à photoélectrode flexible implantable (1, 1') selon la revendication 1, dans lequel la première couche faisant écran à la lumière (104, 104') comprend une première partie transmettant la lumière, et la première partie transmettant de la lumière est configurée pour transmettre le faisceau lumineux (L) à travers elle et pour une sortie dans une direction à distance de la région électroluminescente (N).

3. Dispositif à photoélectrode flexible implantable (1, 1') selon la revendication 2, dans lequel la partie transmettant de la lumière comprend un premier trou d'interconnexion dans la première couche faisant écran à la lumière (104, 104'), et la projection orthographique du premier trou d'interconnexion sur la première couche de support flexible (101, 101') est située dans les limites de la projection orthographique de la région électroluminescente (N) sur la première couche de support flexible (101, 101').

4. Dispositif à photoélectrode flexible implantable (1, 1') selon la revendication 3,
dans lequel le premier trou d'interconnexion est une pluralité, les projections orthographiques d'une partie d'une pluralité de premiers trous d'interconnexion (121, 121') sur la première couche de support flexible (101, 101') se trouvent dans les limites de la projection orthographique de la région électroluminescente (N) sur la première couche de support flexible (101, 101'), et
les projections orthographiques d'une autre partie de la pluralité de premiers trous d'interconnexion (121, 121') sur la première couche de support flexible (101, 101') se trouvent en dehors de la projection orthographique de la région électroluminescente (N) sur la première couche de support flexible (101, 101').

5. Dispositif à photoélectrode flexible implantable (1, 1') selon l'une quelconque des revendications 1 à 4,
dans lequel l'unité électroluminescente (110, 110') est une pluralité, et une pluralité des unités électroluminescentes (110, 110') présente une pluralité de régions électroluminescente (N), et
la première couche faisant écran à la lumière (104, 104') comprend une pluralité de premières parties de transmission de lumière, et la pluralité des premières parties de transmission de lumière est disposée suivant une correspondance d'un-à-un avec la pluralité de régions région électroluminescente (N).

6. Dispositif à photoélectrode flexible implantable (1, 1') selon la revendication 5, dans lequel la pluralité de régions émettant de la lumière (N) est disposée sans espace entre celles-ci, de telle sorte que la pluralité de régions électroluminescentes (N) sont connectées les unes avec les autres.

7. Dispositif à photoélectrode flexible implantable (1, 1') selon l'une quelconque des revendications 1 à 6, comprenant en outre :
un ensemble de fixation (13), l'ensemble de fixation (13) se trouvant sur le corps principal (10, 10') et étant configuré pour fixer le corps principal (10, 10') en place, dans lequel l'ensemble de fixation (13) comprend :
un adhésif (131) ;
un récipient de stockage (132), configuré pour stocker l'adhésif (131) ;
dans lequel le corps principal (10, 10') présente une direction circonférentielle perpendiculaire à une direction d'extension du corps principal (10, 10'), le récipient de stockage (132) étant agencé le long de la direction circonférentielle du corps principal (10, 10').

8. Appareil d'interface cerveau-ordinateur, comprenant le dispositif à photoélectrode flexible implantable (1, 1') selon l'une quelconque des revendications 1 à 7.
